# EUROPÄISCHE PATENTSCHRIFT

(11) **EP 0 796 623 B1**
(45) Veröffentlichungstag und Bekanntmachung des Hinweises auf die Patenterteilung: **25.05.2005**
(21) Anmeldenummer: 97890051.2
(22) Anmeldetag: 18.03.1997
(51) Int. Cl.: A61K 38/48, A61K 38/36, C07K 1/36, A61P 7/04

(54) **Pharmazeutisches Präparat zur Behandlung von Blutgerinnungsstörungen**
Pharmaceutical preparation for the treatment of blood coagulation disorders
Préparation pharmaceutique pour le traitement des maladies de la coagulation sanguine

(30) Priorität: 20.03.1996 AT 51896; 04.09.1996 AT 157396; 20.09.1996 AT 167396
(43) Veröffentlichungstag der Anmeldung: 24.09.1997
(73) Patentinhaber: Baxter Aktiengesellschaft, 1221 Wien (AT)
(72) Erfinder: Turecek, Peter, Dr., 3400 Klosterneuburg/Weidling (AT); Schwarz, Hans-Peter, Prof., 1180 Wien (AT); Eibl, Johann Dr., 1180 Wien (AT)
(74) Vertreter: Alge, Daniel, Mag. Dr. rer.nat.

(56) Entgegenhaltungen:
- EP-A- 0 041 174
- DD-A- 141 262
- DE-A- 3 127 318
- DE-A- 4 430 205

## Beschreibung

Die vorliegende Erfindung betrifft ein pharmazeutisches Präparat, insbesondere zur Behandlung von Blutgerinnungsstörungen, enthaltend Gerinnungsfaktoren, die Bestandteil einer Prothrombinase bzw. Pro-Prothrombinase sind.

Die Prothrombinase ist ein Enzym-Substratkomplex, der sich an einer Phospholipidoberfläche bildet und die Aktivierung von Prothrombin ermöglicht. Die Prothrombinase besteht definitionsgemäß aus dem Faktor II (Prothrombin), dem aktivierten Faktor X (Faktor Xa), den Kofaktor V bzw. Va, Phospholipiden und Calciumionen. Diese Faktoren liegen in vivo als transienter Komplex zur Aktivierung des Prothrombins und Bildung des Thrombins vor.

Eine entsprechende Pro-Prothrombinase ist als Komplex von Faktoren definiert, die zumindest teilweise modifiziert bzw. aktiviert zur Bildung einer Prothrombinase vorliegen. Die Pro-Prothrombinase ist daher als Vorstufe der Prothrombinase und als Komplex zu verstehen, bei welchem eine oder mehrere Komponenten in ihren Vorstufen, als Zymogene oder als Proformen vorliegen und der aufgrund von Affinitäten der Komponenten zueinander gebildet wird.

Haemophilie A entsteht durch einen X-chromosomal-rezessiv-erblichen Mangel an Faktor VIII und manifestiert sich in schweren Blutgerinnungsstörungen. Zur Stillung akuter Blutungen werden meist gerinnungsaktive Plasmaproteinkonzentrate eingesetzt, vorwiegend Faktor VIII-Konzentrate. Bei der klassischen Behandlung von Haemophilie A-Patienten mit Faktor VIII-Präparaten kommt es in rund 20 % der Fälle jedoch zur Bildung von Antikörpern gegen Faktor VIII, welche zu einer Inhibierung der Wirkung der verabreichten Faktor VIII-Präparate führen. Man spricht dann davon, daß ein Patient einen funktionellen Inhibitor gegen Faktor VIII gebildet und eine sogenannte Faktor VIII-Hemmkörperhaemophilie oder erworbene Haemophilie entwickelt hat.

Für die Therapie von Haemophilie A-Patienten mit einer Faktor VIII-Hemmkörperhaemophilie werden gegenwärtig mehrere Methoden angewendet:

### 1.) Behandlung mit hohen Dosen eines Faktor VIII-Präparates:

Damit wird der gegen Faktor VIII gerichtete Antikörper in vivo neutralisiert und der überschüssige Faktor VIII kann seine haemostatische Cofaktoraktivität entfalten. Durch wiederholte Verabreichung über einen längeren Zeitraum wird der betroffene Patient gegen Faktor VIII desensibilisiert und kann anschließend in vielen Fällen der üblichen Faktor VIII-Konzentrattherapie unterzogen werden. Diese Vorgangsweise benötigt außerordentlich große Mengen Faktor VIII, ist zeitaufwendig und kann zu Beginn der Behandlung mit massiven anaphylaktischen Nebenwirkungen behaftet sein.

### 2.) Behandlung von Faktor VIII-Inhibitorpatienten mit Immunglobulinpräparaten, die antiidiotypische Faktor VIII-Antikörper enthalten:

Dieser Therapieweg ist derzeit Gegenstand intensiver Forschung. Für die Effizienz einer solchen Behandlung besteht heute noch keine abschließende Beurteilungsmöglichkeit.

### 3.) Immunadsorption:

Eine weitere aufwendige Methode zur Entfernung der Faktor VIII-Inhibitoren ist die extrakorporale Immunabsorption an entweder Lektine, die Immunglobuline binden (Protein A, Protein G), oder immobilisierten Faktor VIII, an welchem der gegen Faktor VIII gebildete Antikörper gebunden wird. Diese Methode ist für den Patienten aufwendig, weil er dabei an eine Apheresemaschine gebunden ist, kann so wie die vorangegangenen zu keiner Stillung einer akuten Blutung führen und ist außerdem teuer.

### 4.) APCC und Derivate:

Therapie der Wahl ist derzeit die Gabe von aktivierten Prothrombinkomplexkonzentraten (APCC), FEIBA®, AUTOPLEX®, die bei Paenten auch mit hohem Hemmkörpertiter zur Stillung akuter Blutungen eingesetzt werden können (siehe z.B. DE-PS 31 27 318 (2)).

Basierend auf den aktivierten Prothrombinkomplexfaktoren-Konzentraten wurde ein Bestandteil, welcher in diesen u.a. auch enthalten ist, nämlich aktivierter Faktor VIIa, als therapeutisches Prinzip für Faktor VIII-Inhibitorpatienten über den Weg der extrinsischen Gerinnung vorgeschlagen. Ein entsprechendes Präparat, nämlich rekombinanter Faktor VIIa, ist derzeit in klinischer Erprobung (Hedner et al., Transfusion Medicine Reviews 7 (2): 78-83 (1993)). Präklinische Untersuchungen, z.B. an Hunden mit Haemophilie A, haben jedoch gezeigt, daß die Behandlung mit rekombinantem Faktor VIIa ineffektiv ist. Ähnlich ist auch die Erfolgsrate in der Humananwendung nur schwankend. Ein weiterer Nachteil des rekombinanten Faktor VIIa besteht darin, daß dieser aufgrund seiner kurzen in vivo-Halbwertszeit oftmalig in hohen Dosen pro Tag gegeben werden muß, um schwere Blutungen, wenn überhaupt, beherrschen zu können. In solchen Fällen wird versucht, Faktor VIIa gemeinsam mit Antifibrinolytika zu verabreichen, um die Wirkung zu unterstützen.

In der Literatur (z.B. DE 44 16 180 A1) wurde auch vorgeschlagen, eine Kombination von Faktor Xa und Phospholipid therapeutisch zur Behandlung von Haemophilie A-Inhibitorpatienten einzusetzen. In vivo-Versuche an Faktor VIII-defizienten Hunden mit Inhibitor zeigen, daß eine derartige Kombination in einer geeigneten Dosierung in der Lage ist, eine akute Blutung zu stillen. Die therapeutische Breite eines solchen Präparates ist jedoch verhältnismäßig gering, weil die effektive und die thrombogene Dosis, was z.B. im Wessler-Modell im Kaninchen gezeigt werden kann, eng beieinander liegen, da insbesondere Phospholipide ein erhöhtes Thrombogenizitäts-Risiko darstellen.

DE 44 30 205 A betrifft eine Zusammensetzung, die als Gegenmittel für Blut-Antikoagulanzien dient, wobei die Antikoagulanzien ausgewählt sind aus der Gruppe umfassend unter anderem Glykosaminoglykane, sulfatierte Polysaccharide, Hirudine usw., wobei die Zusammenfassung ein Prothrombinkomplex-Konzentrat und wenigstens eine weitere, die Blutgerinnung fördernde Komponente aufweist. Diese Blutgerinnung fördernde Komponente ist beispielsweise Faktor V, Va, XI, XII, XIII oder von Willebrand Faktor.

Die vorliegende Erfindung stellt sich daher zur Aufgabe, die Nachteile der geschilderten Methoden zu vermeiden und ein Therapieprinzip für die Behandlung von Blutgerinnungsstörungen, insbesondere für die Behandlung von Faktor VIII-Hemmkörperhaemophilie, zur Verfügung zu stellen, welches u.a. eine einfache Anwendung, einen effektiven Wirkungseintritt, eine verlängerte Halbwertszeit sowie eine Vermeidung von thrombogenen Nebenwirkungen ermöglicht.

Diese Aufgabe wird erfindungsgemäß mit einer pharmazeutischen Präparation zur Behandlung von Blutgerinnungsstörungen enthaltend mindestens zwei hochgereinigte einzelne Gerinnungsfaktoren ausgewählt aus der Gruppe bestehend aus den Faktoren II, V, Va, X und Xa, die Bestandteile einer Prothrombinase oder Pro-Prothrombinase sind, als aktive Komponenten, insbesondere mit einer Präparation enthaltend gereinigtes Prothrombin und gereinigten Faktor Xa als aktive Komponenten, gelöst, wobei vorzugsweise einer der Faktoren außer Prothrombin aktiviert ist. Die Komponenten sind vorzugsweise zumindest so weit gereinigt, daß sie von endogenen, d.h. aus dem Ausgangsmaterial stammenden, Phospholipiden, aber auch insbesondere von Phospholipidvesikeln, befreit sind.

Damit wird einerseits eine vorzeitige Thrombinbildung unterbunden und die Stabilität der pharmazeutischen Präparation gewährleistet und andererseits das Risiko von thromboembolischen Nebenwirkungen minimiert.

Ein Gemisch bzw. Komplex aus mindestens zwei Bestandteilen der Prothrombinase wird erfindungsgemäß als "partielle Prothrombinase" verstanden.

Neben den Bestandteilen der Prothrombinase bzw. Pro-Prothrombinase sind vorteilhafterweise weitere Faktoren der Blutgerinnung und Fibrinolyse enthalten, um eine attenuierte Wirkung, insbesondere eine Verstärkung, Abschwächung, Beschleunigung oder Verzögerung der Hämostase, zu erzielen. Dementsprechend können Aktivatoren oder Proaktivatoren der Blutgerinnung, darunter Faktoren der intrinsischen oder extrinsischen Blutgerinnung, als Zymogene oder als aktivierte Faktoren, sowie deren Agonisten oder Antagonisten bzw. Inhibitoren weiters enthalten sein. Daneben sind auch die entsprechenden Kombinationen als Präparate möglich, die separat zur Anwendung kommen. Dazu zählt die Kombination mit Fibrinogen, die vor allem für die lokale Applikation geeignet ist.

Entsprechend einer bevorzugten Ausführungsform besteht die pharmazeutische Präparation jedoch im wesentlichen aus der "partiellen Prothrombinase", wobei vorzugsweise die Bestandteile der Prothrombinase bzw. Pro-Prothrombinase als Komplex gebunden vorliegen. Dieser Komplex kann in einfacher Weise gereinigt und behandelt werden, insbesondere zur Inaktivierung von molekularen, mikrobiellen oder viralen Pathogenen chemisch und/oder physikalisch behandelt werden.

Die Faktoren der erfindungsgemäßen pharmazeutischen Präparation sind in einer Form enthalten, die die Aktivierung mindestens eines Faktors ermöglicht bzw. in der mindestens ein Faktor bereits aktiviert ist. Als Faktoren werden vorzugsweise humane Faktoren zugesetzt. Die Faktoren sind in der erfindungsgemäßen pharmazeutischen Präparation ausgewählt, aus der Gruppe bestehend aus den Faktoren II, V, Va, X und Xa.

Als erfindungsgemäße Präparation einer partiellen Prothrombinase bzw. Pro-Prothrombinase werden vorzugsweise Kombinationen der Faktoren II und V bzw. Va sowie X und V bzw. Va zur Verfügung gestellt. Dabei ist besonders bevorzugt, daß die erfindungsgemäße Präparation im wesentlichen aus diesen Kombinationen besteht. Gleichermaßen ist auch eine Pro-Prothrombinase bestehend aus den Faktoren II und X, gegebenenfalls in Kombination mit Faktor V bzw. Va, eine bevorzugte Ausführungsform der vorliegenden Erfindung.

Dabei können native Faktoren, etwa aus Plasma oder einer Plasmafraktion gewonnene Proteine bzw. deren Äquivalente, die beispielsweise von rekombinanten Nukleinsäuren kodiert werden, eingesetzt werden. Des weiteren eignen sich aber auch entsprechende Derivate, welche die modifizierten Proteine oder Fragmente umfassen, solange sie aktivierbar sind bzw. die entsprechende Aktivität aufweisen, um die Generierung von Thrombin zu modulieren.

Die erfindungsgemäße Präparation hat den Vorteil, daß sie trotz der hohen Stabilität in vitro auch in vivo solange stabil ist, bis sich dessen Wirksamkeit durch die Aktivierung von Prothrombin und die Entwicklung von Thrombin am Ort der Wunde bzw. Blutung zeigt. Durch den Kontakt mit den zellulären Bestandteilen des Blutkreislaufes, z.B. Blutzellen oder Gefäßwände, insbesondere phospholipidhältigen Oberflächen wird in situ Thrombin generiert und die Hämostase gefördert. Aufgrund der lokalen Wirksamkeit werden systemische Nebenwirkungen, wie z.B. thromboembolische Komplikationen, vermieden.

Um eine kontrollierte Beeinflussung der Hämostase zu bewirken, ist es bevorzugt, hochgereinigte Faktoren einzusetzen, die von störenden Verunreinigungen, insbesondere von einer Thrombinaktivität, befreit sind. Dafür eignen sich im wesentlichen Faktoren, die durch chromatographische Verfahren, wie der Ionenaustauschchromatographie, Hydrophoben Chromatographie, Affinitätschromatographie und/oder Molekularausschlußchromatographie gereinigt sind. Damit können jeweils spezifische Aktivitäten von mindestens 50% der theoretischen Reinheit, insbesondere mindestens 70%, vorzugsweise mindestens 90% bis zur theoretischen Reinheit für den Einzelfaktor erreicht werden. Dementsprechend ist es auch bevorzugt, Faktoren zu verwenden, die im wesentlichen frei von Denaturierungsprodukten sind und daher als gereinigte aktive Faktoren, Enzyme bzw. aktivierbare Zymogene vorliegen.

Es ist weiters auch bevorzugt eine Behandlung zur Inaktivierung von infektiösen Krankheitserregern vorzunehmen, beispielsweise durch eine Behandlung mit Chemikalien und/oder eine physikalische Behandlung, wie die der Hitzebehandlung, Bestrahlung bzw. der Filtration, insbesondere der Nanofiltration. Gemäß einer bevorzugten Variante sind die Faktoren der erfindungsgemäßen pharmazeutischen Präparation mit Detergentien behandelt, was einerseits zur Inaktivierung von Viren führt und andererseits möglicherweise vorhandene Phospholipide solubilisiert.

Phospholipide können in Präparationen von Blutgerinnungsfaktoren beispielsweise aus Plasma oder aus einer Plasmafraktion bzw. aus einer Zellkultur enthalten sein. Die spezielle Behandlung der Faktoren zur Abtrennung der natürlicherweise vorhandenen Phospholipide umfaßt einerseits die Solubilisierung dieser und andererseits die Abtrennung der Phospholipide durch die erwähnten Reinigungsverfahren.

Obwohl das Präparat in Kombination mit exogenen Phospholipiden eingesetzt werden kann, sind gemäß einer weiteren bevorzugten Variante der efindungsgemäßen Präparate diese frei an zugesetzten Phospholipiden und enthalten weniger als 0,01 mg Phospholipide/E Prothrombin, was aufgrund der möglichen thrombogenen Wirkung von Phospholipiden zu einer weiteren erheblichen Verringerung des Thrombogenizitätsrisikos führt. Gemäß einer besonders bevorzugten Ausführungsform sind die Präparate frei von nachweisbarem Phospholipid.

Entsprechend einer weiteren Ausführungsform enthält die erfindungsgemäße Präparation weiters Magnesiumionen. Diese Ionen wirken kompetitiv zu Calciumionen und können die Calciumionen in der Prothrombinase bzw. Pro-Prothrombinase verdrängen. Damit wird eine vorzeitige Thrombinbildung in einer Lösung der erfindungsgemäßen Präparation unterbunden und damit diese soweit stabilisiert, daß sie in einer Lösung auch nach Stunden stabil bleibt.

Es hat sich herausgestellt, daß die pharmazeutische Präparation sogar als stabile Infusionslösung zur Verfügung gestellt werden kann, vor allem wenn gewährleistet ist, daß sie keine freien Calciumionen enthält. Zur Komplexierung der Calciumionen eignet sich auch ein Gehalt an einem pharmazeutisch akzeptablen Chelatbildner, beispielsweise EDTA, und verwandte Strukturen, wie Citrat.

Das erfindungsgemäße Präparat weist eine FEIBA®-vergleichbare biologische Wirksamkeit in Tiermodellen auf und ist in der Lage, die Gerinnungszeit eines Faktor VIII-Inhibitorplasmas deutlich zu verringern. Es kann die verlängerte Blutungszeit und Blutungsneigung von Faktor VIII-Inhibitorkaninchen und von von Willebrand-Faktor-Inhibitorkaninchen gänzlich normalisieren. Durch das Bereitstellen von gereinigten Blutgerinnungsfaktoren, z.B. von gereinigtem Prothrombin und gereinigtem Faktor Xa, ist die Toxizität des erfindungsgemäßen Präparates im Vergleich zu FEIBA® deutlich reduziert. So erweist sich beispielsweise die effektive Kombination von Faktor Xa und Prothrombin im Wessler-Thrombose-Modell (J.Appl.Phys. 14 (1959), 943-946) als negativ, d.h., es können für diese Kombination auch bei höheren Dosen als bei aktiviertem Prothrombinkomplex keine thrombogenen Effekte im Wessler-Modell nachgewiesen werden.

Bei diesem Thrombogenitätsmodell werden Kaninchen mit Pentobarbital narkotisiert, dann unter zusätzlicher Lokalanästhesie die Vena jugularis freipräpariert und mit losen Ligaturen im Abstand von 2 cm versehen. Schließlich wird die zu testende Substanz, in die der Vena jugularis gegenüberliegende Ohrvene innerhalb von 15 sek injiziert. Nach weiteren 25 sek werden die Ligaturen zugezogen und 10 min gewartet bis das abgebundene Venenstück entnommen und in einer mit Citratpuffer gefüllten Petrischale aufgeschnitten und bewertet werden kann. Die Bewertungskriterien, modifiziert nach Wessler, sind: keine Thrombenbildung = 0, wenige kleine Thromben = 0,5 - 1, wenige mittelgroße und viele kleine Thromben = 2, viele mittelgroße Thromben = 3, wenige große Thromben = 3,5, ein zusammenhängender Thrombus = 4.

Die Komponenten des erfindungsgemäßen pharmazeutischen Präparates sind vorzugsweise bis zu einer derartigen Reinheit aufgereinigt, daß es sogar bei einer Dosis von mindestens 150 E Prothrombin/kg frei an thromboembolischen Nebenwirkungen, ausgedrückt durch einen Score im Wessler-Thrombosemodell von höchstens 3, vorzugsweise höchstens 2, insbesondere weniger als 2 Punkten, ist. Prothrombin ist im erfindungsgemäßen Kombinationspräparat, vorzugsweise in einer spezifischen Aktivität von mindestens 5 E/mg Protein, noch bevorzugter mindestens 6, insbesondere mindestens 7, entsprechend 50, 60 oder 70 % der theoretischen Reinheit, enthalten. Die eingesetzte Faktor X- bzw. Xa-Präparation sollte vorzugsweise eine spezifische Aktivität von mindestens 100 E/mg Protein aufweisen, wobei Faktor Xa vorzugsweise vorwiegend als Faktor Xaβ enthalten ist. Faktor V bzw. Va wird als Cofaktor in einem etwa äquimolaren Verhältnis zum Gerinnungsfaktor der partiellen Prothrombinase bzw. Pro-Prothrombinase eingesetzt. Das Verhältnis beträgt vorzugsweise (0,01-2):1 (mol/mol), am meisten bevorzugt (0,5-2):1.

Das eingesetzte Präparat sollte möglichst frei an Thrombin sein, wobei die Freiheit an Thrombin durch geeignete, vorzugsweise chromogene Tests, nachgewiesen werden kann (z.B. mit dem chromogenen Substrat TH-1 der IMMUNO AG.).

Es zeigte sich, daß, wenn die Gerinnungsfaktoren, wie z.B. Prothrombin und Faktor Xa, im erfindungsgemäßen Präparat als Komplex vorliegen, das Präparat eine erhöhte Stabilität gegenüber herkömmlichen Präparaten aufweist und der Komplex darüber hinaus noch einer weiteren Behandlung zur Reinigung und/oder Inaktivierung von Viren unterzogen werden kann. Bei einer bevorzugten Ausführungsform des erfindungsgemäßen Präparates umfaßt dieses weiters Antithrombin III in stabilisierenden Mengen, gegebenenfalls gemeinsam mit Heparin, wobei jedoch auch bei einem solchen Präparat die Freiheit an thromboembolischen Nebenwirkungen gemäß dem Wessler-Test in Abwesenheit bzw. auch nach Demaskierung des Heparins, d.h. Neutralisation und/oder Abtrennung des Heparins, nachgewiesen werden kann, in dem Sinne, daß der Wert von 3 Punkten nicht erreicht wird.

Ein Komplex, bestehend aus mindestens zwei Gerinnungsfaktoren, die Bestandteile einer Prothrombinase oder Pro-Prothrombinase sind, insbesondere in hochgereinigter Form, hat als "partieller Prothrombinase-Komplex" grundlegende Bedeutung. Die Aktivität eines Präparates auf Basis dieses Komplexes kann durch die Gegenwart von Calciumionen, wie Calciumchlorid, um ein Vielfaches beschleunigt werden. Auch hat sich herausgestellt, daß ein Präparat, enthaltend diesen Komplex und weiters Calciumionen, zur Herstellung eines Reagens für diagnostische Zwecke verwendet werden kann. Ein Reagens, welches weiters Thrombinaktivität und gegebenenfalls Phospholipide enthält, ist beispielsweise zur Bestimmung der Faktor V-Cofaktoraktivität geeignet. Ein diagnostisches Verfahren unter Verwendung dieses Reagens mit bzw. ohne aktiviertem Protein C, einem proteolytischen Inaktivator von Faktor V, ermöglicht darüberhinaus noch die Abschätzung eines Ausmaßes einer Inaktivierung von Faktor V bzw. eine mutationsbedingte Resistenz gegenüber aktiviertem Protein C.

Vorzugsweise ist das Prothrombin bis zu einem Grad aufgereinigt, daß es sogar bei einer Dosis von mindestens 150 E Prothrombin/kg frei an thromboembolischen Nebenwirkungen, ausgedrückt durch einen Score im Wessler-Thrombosemodell von höchstens 3, vorzugs weise höchstens 2, insbesondere weniger als 2 Punkten ist.

Das erfindungsgemäße Kombinationspräparat enthält bevorzugterweise weniger als 0,1 E Faktor VIII:C oder Faktor VIII:Ag/E Prothrombin bzw. weniger als 0,1 E Faktor IX/E Prothrombin bzw. weniger als 0,1 E Faktor X/E Pro thrombin. Dadurch kann die unerwünschte Bildung von bzw. Reaktion mit Antikörpern gegen diese Proteine effizienter hintan gehalten und das Risiko von Nebenwirkungen verringert werden.

Die Dosierung der erfindungsgemäßen Präparate orientiert sich an der Dosierung der äquivalenten Komponenten in FEIBA®. Die Factor Eight Inhibitor Bypassing-Aktivität (FEIBA) wird definiert als jene Aktivität einer derartigen Präparation, die die Gerinnungszeit eines Faktor VIII-Inhibitorplasmas in einem Gerinnungstest, wie er in AT 350726 beschrieben wird, auf 50 % des Leerwertes reduziert. Die Vorteile der erfindungsgemäßen Präparate im Vergleich zu FEIBA liegen bedingt durch die hohe Reinheit der Komponenten in einer reduzierten Belastung des Patienten mit Plasmaproteinen. Insbesondere ist durch das Fehlen von Faktor VIII:Ag die anaphylaktische Nebenwirkung ausgeschlossen. Es ist daher möglich, die erfindungsgemäßen Präparate so hoch zu konzentrieren, daß eine Dosis verabreicht werden kann, welche beispielsweise mindestens 50 E Prothrombin/kg Körpergewicht umfaßt, wobei diese Dosis auf Grund der Nebenwirkungsfreiheit erstmalig bei dieser Art von Präparaten sogar in einer Bolusinjektion verabreicht werden kann, womit die ansonsten langwierige Verabreichung derart hoher Dosen vermieden werden kann.

Üblicherweise werden in den erfindungsgemäßen Präparaten, eine Dosis von beispielsweise 50 bis 150 E Prothrombin/kg Körpergewicht verabreicht, wobei die maximalen Dosen jedoch auch weit über den 150 E/kg Körpergewicht liegen können (z.B. bis 300 bzw. bis 500), ohne daß es zu thromboembolischen Nebenwirkungen kommen kann.

Gegenstand der vorliegenden Erfindung sind daher auch Darreichungsformen der erfindungsgemäßen pharmazeutischen Präparate, welche eine Dosis von mindestens 50 E Prothrombin/kg Körpergewicht, vorzugsweise zwischen 50 und 500 E/kg Körpergewicht, umfassen.

Diese Darreichungsformen können bereits zur direkten Verabreichung vorgesehene Ampullen, Spritzen, oder ähnliche direkt oder indirekt applizierbare Formen sein. Dazu zählen Behälter geeignet zur Infusion, intramuskulären bzw. subkutanen Anwendung oder Sets, bestehend aus Behälter mit den Wirkstoffen als Lyophilisat und einem Behälter mit einer pharmazeutisch akzeptablen Lösung, geeignet zur Rekonstitution des Lyophilisats. Üblicherweise ent-hält die pharmazeutisch akzeptable Lösung bzw. das pharmazeutische Präparat Salze, Konservierungsstoffe, Puffer und dgl. in einer wässerigen Lösung (siehe Remington's Pharmaceutical Sciences, 15. Aufl., Easton: Mack Publishing Co., S. 1405-1412 und 1461-1487 (1975) und The National Formulary XIV., 14. Aufl., Washington: American Pharmaceutical Association (1975)). Beispielhaft für nicht-wässerige Lösungen sind Propylenglykol, Polyethylenglykol, pflanzliche Öle und injizierbare organische Ester, wie Ethyloleat. Wässerige Träger sind beispielsweise Wasser, gegebenenfalls mit Alkohol gemischt, Salzlösungen (NaCl), Ringer's Dextrose, etc.. Als Konservierungsstoffe können antimikrobielle Substanzen, Antioxidantien, Chelatbildner oder Inertgase verwendet werden.

Die erfindungsgemäßen Präparate eignen sich auch zur lokalen Behandlung, wobei Applikationsformen gewählt werden, die am Ort einer Blutung wirksam werden. Dazu zählen Feststoffe oder Flüssigkeiten, vorzugsweise in Form eines Puders, Pflasters bzw. Wundauflage, Salben, Suppositorien, Kapseln, insbesondere magensaftresistente Kapseln, aber auch Tropfen oder Sprays.

Die eingesetzten Gerinnungsfaktoren können sowohl plasmatischen Ursprungs als auch durch rekombinante DNA-Technologie hergestellte Proteine sein. Wesentlich ist in beiden Fällen, daß sie in gereinigter Form, insbesondere in einer von endogenen und exogenen Phospholipiden befreiter Form im pharmazeutischen Präparat vorliegen.

Bevorzugterweise werden die erfindungsgemäßen pharmazeutischen Präparate in lyophilisierter Form zur Verfügung gestellt, was die bekannten Transport-, Lagerungs- und Applikationsvorteile mit sich bringt. Als Rekonstitutionslösung bietet sich eine pharmazeutisch akzeptable Lösung an, die gegebenenfalls ATIII bzw. Heparin enthält. Durch den hohen Reinheitsgrad der Komponenten der erfindungsgemäßen Präparate können diese nach geringer Lösungszeit bei Raumtemperatur, vorzugsweise unter 5 min, insbesondere unter 1 min, zu einer optisch klaren Lösung mit beispielsweise mindestens 10 E Prothrombin/ml rekonstituiert werden, wobei sogar Konzentrationen von bis zu 200 E Prothrombin/ml Lösung erreicht werden können. Eine optisch klare Lösung ist dabei definiert durch ein Maximum der Extinktion bei 600 nm von 0,1 (für eine Lösung mit mindestens 5 Gew.% Proteingehalt, bei einer Schichtdicke von 1 cm), bezogen auf die reine (Puffer-) Lösung als Referenz. Alternativ dazu gilt auch eine Lösung mit weniger als 70 Light Scattering Units (LSU), ermittelt durch Messung im Nephelometer bei 340 nm und einer Schichtdicke von 1 cm als klar.

Die erfindungsgemäßen Präparate sind im Gegensatz zu den bisher bekannten Präparaten zur Behandlung von Blutgerinnungsstörungen außerordentlich stabil, d.h., sie können für eine längere Zeitperiode vor der Verabreichung stehen gelassen werden. Beispielsspielsweise sollte FEIBA® im verabreichungsfertigen Zustand gemäß der Produktinformation nicht länger als 1 Stunde stehen gelassen werden, wohingegen die erfindungsgemäßen Präparate als gebrauchsfertige Lösung auch während eines Zeitraums von 3 Stunden oder länger bei Raumtemperatur keinerlei Gerinnungsaktivierung bzw. Thrombogeniziät zeigen, weshalb die erfindungsgemäßen Präparate auch als Infusionslösung zur Verfügung gestellt werden können, die auch über einen Zeitraum von mehreren Stunden verabreicht werden kann. Aus den gleichen Gründen ist es auch möglich, die erfindungsgemäßen Präparate über einen längeren Zeitraum als Infusionslösung zu verabreichen. Es hat sich aber gezeigt, daß in Bezug auf die thromboembolischen Nebenwirkungen kein wesentlicher Unterschied zwischen einer Bolusinjektion und einer langsamen Infusion mit den erfindungsgemäßen Präparaten auftritt.

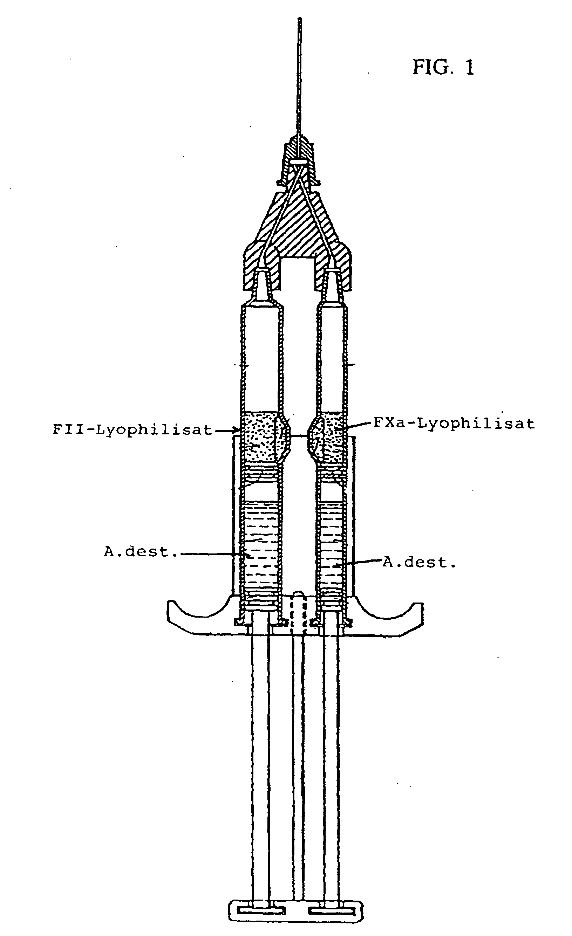

Gemäß einer bevorzugten Ausführungsform der vorliegenden Erfindung werden die pharmazeutischen Präparate in geeigneten Applikationsvorrichtungen vorgegeben, vorzugsweise als Lyophilisat in Spritzen, welche eine in situ-Rekonstitution mit einer pharmazeutisch akzeptablen Lösung erlauben. Beim Kombinationspräparat empfiehlt sich eine Applikationsvorrichtung, wie in Fig. 1 gezeigt, worin die Lyophilisate von beispielsweise Prothrombin und Faktor Xa vorzugsweise getrennt aufbewahrt werden und bei Bedarf nach einer in situ-Rekonstitution mittels einer Doppelkammerspritze verabreicht werden können.

Die pharmazeutischen Präparate gemäß der vorliegenden Erfindung können, insbesondere wenn sie aus plasmatischen Proteinen oder Zellkulturen gewonnen werden, einer oder mehreren Virus-Inaktivierungsbehandlungen bzw. Behandlungen zur Virus-Abreicherung unterzogen werden, beispielsweise einer chemischen oder chemisch-physikalischen Behandlung, einer Hitze- und/oder Detergensbehandlung gemäß der EP 0 159 311, der EP 0 519 901 oder der EP 0 674 531 oder einer physikalischen Behandlung, wie die der Nanofiltration.

Die erfindungsgemäßen Präparate ermöglichen eine sichere und einfache Behandlung von Blutgerinnungsstörungen, bei welcher ein effektiver Wirkungseintritt innerhalb kürzester Zeit beobachtet werden kann.

Es zeigt sich, daß der effektive Wirkungseintritt bei der Gabe des Komplexes von Faktor II und Faktor Xa schneller erfolgt, als für ein Präparat, welches ausschließlich Faktor II enthält.

Zur Behandlung einer Blutungskomplikation bei einem Patienten mit Hemmkörperhaemophilie ist es daher vorteilhaft, das Blutungsereignis initial durch die Gabe des Faktor II/Xa-Komplexes zu behandeln und somit zu einer schnellen Blutstillung zu kommen und die Therapie zur Vermeidung weiterer Blutungen mit Erhaltungsdosen eines Präparates, welches ausschließlich Faktor II ent hält, fortzusetzen.

Darüberhinaus ermöglichen die lange Halbwertszeit der erfindungsgemäßen Präparate und deren Freiheit an thrombogenen Neben wirkungen bzw. das Ausbleiben einer anaphylaktischen Reaktion, welche zur Verstärkung des Inhibitortiters führt, eine gegenüber bekannten Verfahren erheblich verbesserte Behandlung des Patienten mit Blutgerinnungsstörungen. Der Patient kann durch die hohe Konzentration bzw. Dosis des erfindungsgemäßen Präparates ein Wirkstoffdepot erhalten, was den Bedarf an oftmaligen Behandlungen herabsetzt. Der Patient kann also auch über einen längeren Zeitraum von mehreren Tagen ohne Behandlung bleiben und sich gegebenenfalls durch Selbstinjektion, eventuell subkutan, ambulant behandeln.

Gemäß einem weiteren Aspekt betrifft die vorliegende Erfindung die Verwendung von gereinigten Prothrombinase-Faktoren, insbesondere von gereinigtem Prothrombin und gereinigtem Faktor Xa zur Herstellung eines pharmazeutischen Präparates zur Etablierung von supranormalen Prothrombin-Konzentrationen im Blut eines Patienten. bzw. zur Etablierung von normalen Prothrombin-Konzentrationen im Blut bei Zuständen mit erniedrigtem Prothrombin-Level.

Es zeigte sich, daß mit den erfindungsgemäßen Präparaten solche supranormalen Faktor II-Levels sogar permanent, d.h. über länge re Zeiträume erhalten werden können, was einesteils darauf zu rückzuführen ist, daß Prothrombin eine für Blutgerinnungsfaktoren sehr hohe Halbwertszeit als Medikament aufweist und anderer seits auch in der Nebenwirkungsfreiheit der erfindungsgemäßen Präparationen begründet ist, so daß sogar eine subkutane Verabreichung etwa durch Depotverabreichung möglich ist. Die supra normalen Konzentrationen von Prothrombin, die im Blut durch Gabe von gereinigtem Prothrombin und gegebenenfalls gereinigtem Faktor Xa bzw. anderen Prothrombinase-Faktoren möglich sind, betragen zumindest 150 %, vorzugsweise sogar mindestens 200 %, entsprechend einer Aktivität von mindestens 1,5 E Prothrombin/ml Blut, vorzugsweise mindestens 2,0 bis zu 10 E/ml.

Schließlich betrifft die Erfindung auch die Verwendung von gereinigten Prothrombinase-Faktoren, insbesondere von gereinigtem Prothrombin und gereinigtem Faktor Xa, zur Herstellung eines pharmazeutischen Präparates zur Behandlung von Faktor VIII-Inhibitorzuständen, Haemophilie A oder B und von Willebrand-Krankheit. Es hat sich gezeigt, daß in Tiermodellen für alle diese Indikationen mit den erfindungsgemäßen Präparaten eine rasche, effiziente und nebenwirkungsfreie Behandlung mög lich ist.

Die erfindungsgemäßen Präparate werden bevorzugt in einer Lösung mit physiologischem pH zur Verfügung gestellt, welche vorzugsweise keine freien Calciumionen enthalten. Es ist aber auch möglich, einen beispielsweise vom Faktor Xa-Aktivitäts-Optimum entfernten sauren Puffer im Bereich von pH 4,5-6,5, vorzugsweise 5-6, zu verwenden, mit welchem die Aktivierung von Prothrombin hintangehalten und somit die Stabilität des Kombinationspräparates nochmals gesteigert werden kann. Es versteht sich, daß sämtliche für Faktor II, V, Va, X und Xa geeigneten pharmazeutischen Zusatzstoffe und Lösungen zur verabreichungsfertigen Herstellung der erfindungsgemäßen Präparate verwendet werden können.

Weiters hat sich überraschenderweise herausgestellt, daß mit den erfindungsgemäßen Präparationen auch bei nicht-haemophilen Patienten akute Blutungen, eine gesteigerte Blutungsneigung bzw. ein erhöhtes Blutungsrisiko effektiv behandelt werden können. Die erfindungsgemäßen Präparationen sind neben den bereits genannten Indikationen der verschiedenen Formen der Haemophilie, d.h. Haemophilie A und B sowie Inhibitorhaemophilie, auch bei nicht-haemophilenn Patienten anwendbar. Zu den nicht-haemophilen Patienten zählen auch jene, die Blutgerinnungsstörungen aufgrund von Inhibitoren gegen Blutfaktoren aufweisen, welche nicht Faktor VIII oder Faktor IX sind. Weiters können Patienten behandelt werden, die eine gestörte Thrombingeneration zeigen, welche durch das Fehlen oder einen funktionellen Defekt eines oder mehrerer Faktoren der extrinsischen oder intrinsischen Gerinnung oder bei Bildung von Antikörpern gegen einen oder mehreren die ser Faktoren oder durch Fehlen des zellulären Rezeptors für einen oder mehreren dieser Faktoren verursacht ist. Nach Gabe einer erfindungsgemäßen Präparation kommt es in vivo gegebenenbenenfalls zu einer gerinnungsfördernden Wirkung, wodurch eine Behandlung, nämlich prophylaktische bzw. therapeutische Verabreichung möglich wird.

Die vorliegende Erfindung betrifft somit gemäß einem weiteren Aspekt die Verwendung von mindestens 2 Gerinnungsfaktoren, die Bestandteile einer Prothrombinase bzw. Pro-Prothrombinase und frei von Phospholipiden sind, wobei die Gerinnungsfaktoren ausgesucht sind aus der Gruppe bestehend aus den Faktoren II, V, Va, X und Xa, bzw. die Verwendung der erfindungsgemäßen Präparation, zur Herstellung einer pharmazeutischen Präparation zur Behandlung von akuten Blutungen, einer gesteigerten Blutungsneigung bzw. einem erhöhten Blutungsrisiko bei nicht-haemophilen Patienten.

Im speziellen können Zustände aufgrund eines gestörten Aggregationsverhaltens von Blutplättchen oder Thrombopathien, z.B. Storage-Pool Defekte, oder bedingt durch Mangel- oder Dysfunktion von plättchenassoziierten Proteinen, aber auch Blutungszustände bedingt durch Plättchenmangel (Thrombozytopänie) behandelt werden. Eine Nebenwirkung einer Antikoagulantien-Therapie liegt in der Heparin-induzierten Thrombozytopänie, die ebenfalls eine Indikation für die erfindungsgemäße Präparation darstellt.

Gemäß einer bevorzugten Ausführungsform betrifft die vorliegende Erfindung daher die Verwendung von mindestens 2 Gerinnungsfaktoren die Bestandteile einer Prothrombinase bzw. Pro-Prothrombinase und frei von Phospholipiden sind, wobei die Gerinnungsfaktoren ausgesucht sind aus der Gruppe bestehend aus den Faktoren II, V, Va, X und Xa, zur Herstellung einer pharmazeutischen Präparation zur Behandlung von Blutungen aufgrund einer Thrombozytopänie, insbesondere zur Behandlung einer Heparin-induzierten Thrombozytopänie.

In diesem Zusammenhang ist es von Vorteil, daß die nach der erfindungsgemäßen Verwendung hergestellte pharmazeutische Präparation unter Umständen eine primäre hämostatische Aktivität aufweist. Die hergestellte pharmazeutische Präparation kann durch die Kombination mit Proteinen mit primärer hämostatische Aktivität verstärkt werden. Dafür eignet sich vor allem ein von Willebrand Faktor-Protein bzw. eine Fraktion des von Willebrand Faktors mit einer definierten Kollagenbindungsaktivität.

Eine weitere Indikation zur Behandlung von Patienten mit Blutgerinnungsstörungen liegt in der Verhinderung bzw. Behandlung von Blutungen, die im Zusammenhang mit der von Willebrand's Disease auftreten. Diese Krankheit mit und ohne der Prävalenz von Gerinnungsfaktorinhibitoren bringt eine erhöhte Blutungsneigung oder ein Blutungsrisiko mit sich und führt in vielen Fällen zur schwer kontrollierbaren Blutungen. Mit Hilfe der erfindungsgemäßen Präparation ist es möglich, eine derartige Blutung rasch zum Stillstand zu bringen.

Somit wird erfindungsgemäß ein Set zur Behandlung von Patienten mit Blutgerinnungsstörungen zur Verfügung gestellt, welches die folgenden Komponenten umfaßt:
a) eine pharmazeutische Präparation enthaltend mindestens 2 Gerinnungsfaktoren, die Bestandteile einer Prothrombinase bzw. Pro-Prothrombinase und frei von Phospholipiden sind, wobei die Gerinnungsfaktoren ausgesucht sind aus der Gruppe bestehend aus den Faktoren II, V, Va, X und Xa, und
b) ein Protein mit primärer hämostatischer Aktivität, insbesondere vWF.

Schwer kontrollierbare Blutungen können fallweise als Nebenwirkung einer Therapie mit synthetischen, halbsynthetischen und biologischen Gerinnungsinhibitoren bzw. Antikoagulantien oder Thrombozytenfunktionshemmern auftreten. Diese Substanzen greifen unmittelbar bzw. mittelbar in das Gerinnungssystem ein und können den natürlichen Gerinnungsvorgang in unerwünschter Weise stören. Es besteht daher Bedarf für Antagonisten für diese Substanzen. Im Stand der Technik ist die Verwendung eines Prothrombinkomplexes oder einer FEIBA®-Präparation zur Behandlung von Blutungen, die durch eine Antikoagulantien-Therapie verursacht sind, bekannt. Siehe dazu Irani M S et al., The American Journal of Cardiology, Vol. 75, Feb. 15, 1995, p 422; Fareed J et al., Haemostasis 1991, Vol. 21 (suppl. 1) p 64-72; und Fareed J et al., Seminars in Thrombosis and Hemostasis 1991, Vol. 17, No. 2, p 137.

Gemäß einer weiteren Ausführungsform der vorliegenden Erfindung werden die genannten Gerinnungsfaktoren zur Herstellung einer erfindungsgemäßen pharmazeutischen Präparation zur Behandlung von Blutungen im Rahmen einer Antikoagulantien-Therapie, insbesondere zur Herstellung einer pharmazeutischen Präparation als Antidot für einen Gerinnungsinhibitor bzw. für ein Antikoagulans oder für einen Thrombozytenfunktionshemmer verwendet. Wesentlich ist dabei, daß ein mit der Gegenwart von Phospholipiden verbundenes Thromboserisiko vermieden wird. Gerade bei Patienten, die mit Antikoagulantien therapiert werden, besteht ein erhöhtes Thromboserisiko. Erfindungsgemäß kann jedoch auf die Verwendung der Phospholipide verzichtet werden, wodurch überraschenderweise die Antidotwirkung noch spezifischer wird.

Die erfindungsgemäße Verwendung betrifft vor allem die Antagonisierung eines Gerinnungsinhibitors bzw. Antikoagulans, welche den Faktor Xa oder Thrombin mittelbar oder unmittelbar inhibieren. Erfindungsgemäß wird vorzugsweise eine Substanz antagonisiert, welche ausgewählt ist aus der Gruppe bestehend aus APAP ((2S)-2-[4-[[(3S)-1-Acetimidyl-3-pyrrolidinyl]oxy]phenyl]-3-(7-amidino-2-naphthyl)-propionsäurehydrochlorid-pentahydrat), Benzamidinderivat, Hirudin, Heparin, Heparinanaloga, insbesondere Pentasaccharide, AT III-Heparinkomplex, AT III, Antistasin, "Tick-Anticoagulant Peptide", inaktive Gerinnungsfaktoren, insbesondere "active site" inhibierte Gerinnungsfaktoren, TFPI, kompetitive Liganden für Thrombozytenmembranoberflächenrezeptoren, insbesondere Antikörper gegen GP IIb/IIIa, und deren gentechnisch oder synthetisch hergestellten Analoga, insbesondere Peptide, sowie orale Antikoagulantien. Zu den inaktiven Gerinnungsfaktoren zählen vor allem derivatisierte, mutierte, fragmentierte, chemisch oder physikalisch inaktivierte bzw. inhibierte Faktoren der intrinsischen bzw. extrinsischen Blutgerinnung, welche in kompetitiver Weise mit dem nativen Faktor in Wechselwirkung treten können.

Die Antagonisierung eines Thrombozytenfunktionshemmers ist vor allem im Fall von Ticlopidin oder Acetylsalicylsäure indiziert.

Akute Blutungen sind vor allem im Bereich des Gehirns sehr kritisch. Deshalb besteht der Bedarf der Verhinderung bzw. Behandlung von intracraniellen Blutungen, z.B. intraventrikuläre Hämorrhagie (IVH). Ein erhöhtes Risiko für intracranielle Blutungen besteht vor allem bei Patienten mit einer Schädigung der Blutgefäße bzw. bei gestörter Thrombingenerierung. Die erfindungsgemäße Präparation ist auch speziell für diese Indikation geeignet, wobei vor allem die verbesserte Behandlung von Frühgeburten ermöglicht wird.

Im Rahmen der erfindungsgemäßen Verwendung zu den genannten Indikationen werden jeweils die entsprechenden Sets zur Verfügung gestellt, welche als eine Komponente die erfindungsgemäße pharmazeutische Präparation enthält. Weiters können Substanzen, die den hämostatischen Effekt verstärken, wie z.B. ein Protein mit primärer hämostatischer Aktivität, insbesondere vWF enthalten sein. Daneben enthält ein Set zur Antikoagulantien-Therapie natürlich das entsprechende Antikoagulans, und die erfindungsgemäße pharmazeutische Präparation als Antidot.

Die Erfindung wird anhand der folgenden Beispiele und der dazugehörigen Zeichnungsfiguren, auf die sie jedoch nicht eingeschränkt sein soll, näher erläutert.

Es zeigen:
Fig. 1 und Fig. 2 mögliche Darreichungsformen der erfindungsgemäßen Präparate;

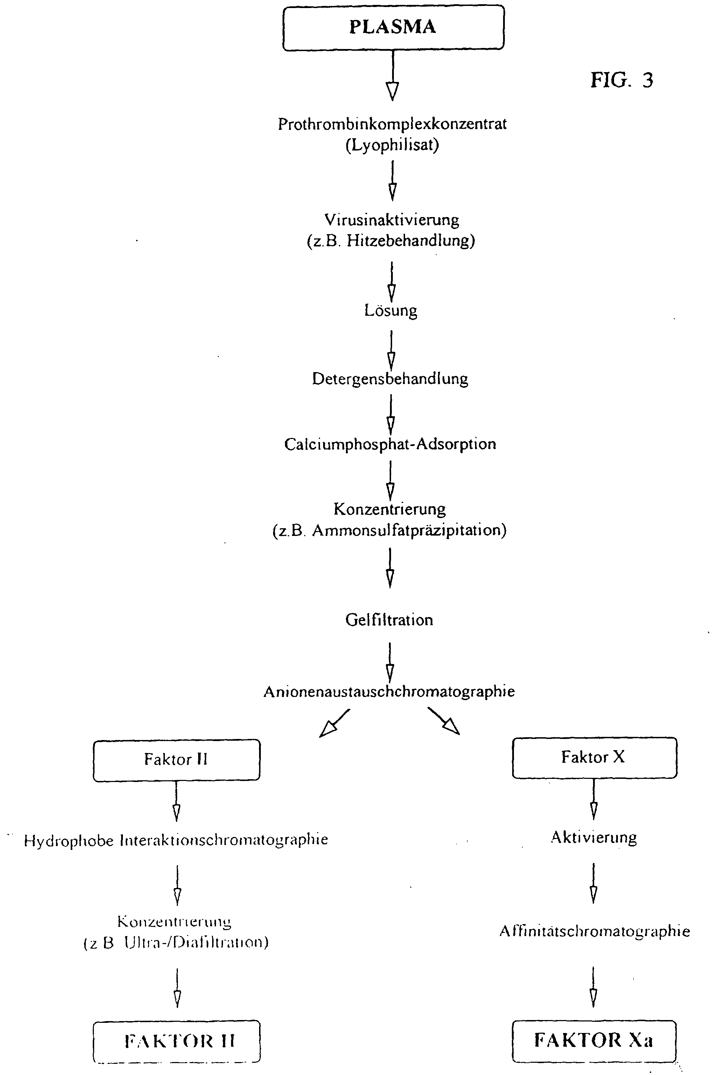

Fig. 3 das Flußdiagramm einer Ausführungsform des Herstellungsverfahrens;
Fig. 4 den Nachweis des partiellen Prothrombinasekomplexes;
Fig. 5 die spektroskopische Analyse eines Beispieles der erfindungsgemäßen Präparate (A) im Vergleich zu Standardpräparaten [aktivierter Prothrombinkomplex (B), Prothrombinkomplex (C)];
Fig. 6 die in vivo-Wirkung einer Faktor II-Präparation auf Faktor VIII-Inhibitorkaninchen;
Fig. 7 und Fig. 8 die in vivo-Wirkung der erfindungsgemäßen Präparate im von Willebrand Faktor/Faktor VIII-Inhibitormodell.

### BEISPIEL 1:

### Herstellung von Faktor X und Faktor II aus einer virusinaktivierten Plasmafraktion mittels Ionenaustauschchromatographie.

Eine lyophilisierte Prothrombinkomplexfaktorenpräparation, welche die Faktoren II, IX, X sowie Protein C und Protein S enthielt, wurde nach der Methode von Brummelhuis, H.G.J., Preparation of the Prothrombincomplex. In: Methods of Plasma Protein Fractionation, Curling, J.M. ed., 117-128, Academic Press, New York, (1980), hergestellt und zur Virusinaktivierung nach EP 159 311 hitzebehandelt. Entsprechend wurde das Lyophilisat (1000 E Faktor X/g, 1200 E Faktor II/g) in destilliertem Wasser gelöst, sodaß dieses 50 000 E Faktor X/l enthielt, und auf pH 7,0 eingestellt. Nach Zusatz von 12 % (v/v) TWEEN 80 wurde 1 Stunde bei Raumtemperatur gerührt. Anschließend wurde mit einem 20 mM Tris-HCl-Puffer, pH 7,0, 1:5 verdünnt und die Prothrombinkomplexproteinfraktion an Calciumphosphat [Ca₃(PO₄)₂] in einer Konzentration von 30 g Ca₃(PO₄)₂ pro l Prothrombinkomplexlösung durch einstündiges Rühren bei Raumtemperatur adsorbiert. Anschließend wurde die feste Phase durch Zentrifugation, 20 Minuten bei 5000 rpm, abgetrennt und der Niederschlag zweimal mit 20 mM Tris-HCl-Puffer, pH 7,0, enthaltend 10 % Ammoniumsulfat, durch Resuspension und erneute Zentrifugation gewaschen. Eine dritte Waschung wurde mit 20 mM Tris-HCl-Puffer, pH 7,0, enthaltend 150 mM NaCl in analoger Weise durchgeführt. Die Elution der Prothrombinkomplexfraktion erfolgte mit 1 M Natriumphosphatlösung, pH 7,0, wobei 25 ml dieser Lösung pro g Calciumphosphat 1 Stunde bei Raumtemperatur gerührt und anschließend der verbleibende Niederschlag durch Zentrifugation wie oben abgetrennt wurde. Der Überstand wurde anschließend einer Ammoniumsulfatfällung mit 366 g Ammoniumsulfat pro l 15 h bei 4°C unter Rühren unterzogen. Das Präzipitat, enthaltend die Prothrombinkomplexfraktion, wurde wie oben abzentrifugiert. Der Niederschlag wurde in einem 25 mM Trinatriumcitratdihydrat-Puffer, enthaltend 100 mM NaCl, 1 mM Benzamidinhydrochlorid, pH 6,0, aufgenommen und auf einer Säule, gefüllt mit Sephadex® G-25, bei 4°C mit einem linearen Fluß von 1 cm/min gegen 25 mM Trinatriumcitratdihydrat-Puffer, enthaltend 100 mM NaCl und 1 mM Benzamidinhydrochlorid, pH 6,0, umgepuffert, um das Ammoniumsulfat abzutrennen. Dabei wurde im Eluatstrom die UV-Absorption bei 280 nm und die elektrische Leitfähigkeit gemessen. Die Protein enthaltenden Fraktionen wurden vereinigt und anschließend einer Ionenaustauschchromatographie über DEAE-Sepharose FF®, Fa. Pharmacia, unterzogen. Die Fraktionen wurden auf einer Säule (Innendurchmesser : Gelbetthöhe = 1 : 1,3) mit einem Gelvolumen von 8,2 l, 0,55 g Protein/l Gel, bei einem linearen Fluß von 0,36 cm/min aufgetragen. Die Chromatographie erfolgte bei 22°C. Vor Auftrag der Proteine war die Säule mit einem 25 mM Trinatriumcitratdihydrat-Puffer, enthaltend 100 mM NaCl, 1 mM Benzamidinhydrochlorid, pH 6,0, äquilibriert worden. Die Elution der Proteinfraktionen erfolgte in mehreren Stufen mit einem Puffer 1 (25 mM Trinatriumcitratdihydrat, 1 mM Benzamidinhydrochlorid, 245 mM NaCl, pH 6,0), Puffer 2 (25 mM Trinatriumcitratdihydrat, 1 mM Benzamidinhydrochlorid, 270 mM NaCl, pH 6,0) und einem Puffer 3 (25 mM Trinatriumcitratdihydrat, 1 mM Benzamidinhydrochlorid, 400 mM NaCl, pH 6,0). Die Elution mit Puffer 1 wurde mit 2,4 Säulenvolumen durchgeführt, dabei wurde Inertprotein abgetrennt. Die Elution wurde mit 5,6 Säulenvolumen mit Puffer 2 durchgeführt, wobei hier Fraktionen gesammelt wurden, die auf Gehalt von Faktor II, Faktor X, Protein C und Faktor IX analysiert wurden. Die Faktor X enthaltenden Fraktionen, die frei von Faktor II, IX und Protein C wurden vereinigt. Durch Elution mit Puffer 3 (1,9 Säulenvolumen) wurde Faktor II desorbiert, wobei wieder Fraktionen gesammelt und auf den Gehalt an Faktor X, Faktor IX und Faktor II untersucht wurde. Die Faktor II enthaltenden Fraktionen wurden gepoolt. Sowohl der Faktor II, als auch der Faktor X enthaltende Pool konnten gegebenenfalls durch Zusatz von 1 M KSCN und Inkubation bei 22°C für mehrere Stunden einer zusätzlichen Behandlung zur Inaktivierung pathogener Verunreinigungen unterworfen werden.

### BEISPIEL 2 :

### Reinigung von Faktor II mittels hydrophober Interaktionschromatographie (Zwischenprodukt)

Der in Beispiel 1 gewonnene Faktor II-Pool wurde durch Zugabe von Natriumchlorid auf 1,8 M NaCl eingestellt und der pH-Wert auf pH 7,0 korrigiert. Diese Lösung wurde anschließend durch hydrophobe Interaktion an ein Gel, Phenylsepharose High Performance®, Fa. Pharmacia, adsorbiert, wobei 3 g Protein/l Gel gebunden wurden. In einer Säule mit einem Verhältnis Innendurchmesser : Gelbetthöhe = 1 : 1,9 wurde bei einem linearen Fluß von 0,25 cm/min die Proteinfraktion adsorbiert und anschließend durch Waschen mit einem Puffer (25 mM Tris-HCl, 3 M NaCl, pH 7,4) vom Inertprotein befreit. Durch Gradientenelution mit 11,5 Säulenvolumina von 3 M - 0,9 M NaCl unter gleichzeitiger Sammlung von Fraktionen wurde Faktor II von der Säule eluiert, wobei jene Fraktionen gepoolt wurden, die Faktor II-Aktivität enthielten, aber frei von Faktor X und Faktor IX waren. Die gesammelten Faktor II-Fraktionen wurden anschließend durch Ultra-/Diafiltration über eine Ultrafiltrationsmembran mit einem cut-off von 30 kD zehnfach aufkonzentriert und gegen einen Puffer enthaltend 4 g Trinatriumcitratdihydrat/l, 8 g NaCl/l, pH 7,0, umgepuffert. Eine so hergestellte Faktor II-Präparation wies eine spezifische Aktivität von 6,9 E/mg Protein auf. Die Bestimmung der Faktor II-Aktivität erfolgte mit der 1-Stufen-Methode, basierend auf der Thromboplastinzeit, unter Verwendung eines Faktor II-Mangelplasmas gegen den Internationalen Faktor II-Standard unter Verwendung der Reagenskombination von IMMUNO, Wien. Andere Gerinnungsfaktoren waren in Gerinnungsanalysen in Spuren oder gar nicht mehr nachweisbar (Faktor VII < 0,00002 E/E Faktor II, Faktor IX 0,0002 E/E Faktor II, Faktor X 0,004 E/E Faktor II, Protein C 0,003 E/E Faktor II und Faktor VIII < 0,0002 E/E Faktor II).

### BEISPIEL 3 :

### Reinigung von Faktor II mittels hydrophober Interaktionschromatographie und Hydroxylapatitchromatographie (Zwischenprodukt)

Als alternatives Herstellungsverfahren für einen hochgereinigten Faktor II wurde auch ein Verfahren verwendet, bei dem aus einem lyophilisierten Prothrombinkomplexfaktorenpräparat (siehe Beispiel 1) durch hydrophobe Chromatographie zuerst Faktor IX abgetrennt, anschließend Faktor II isoliert und dieser dann durch Chromatographie an Hydroxylapatit hochgereinigt wurde.

Die Prothrombinkomplexfaktorenpräparation wurde wie in Beispiel 1 gelöst und mit Detergens 1 h bei Raumtemperatur inkubiert. Anschließend wurde durch Ionenaustauschchromatographie auf DEAE-Sepharose FF®, Fa. Pharmacia, wie in Beispiel 1, eine Faktor II, IX und X-hältige Fraktion isoliert. Aus dieser wurde anschließend durch Interaktion mit Butyl-Toyopearl®, Fa. Toso Haas, die Faktor IX-enthaltende Fraktion entfernt. Der Adsorptionsüberstand wurde anschließend an Phenyl-Sepharose High Performance®, Fa. Pharmacia, durch eine weitere hydrophobe Interaktionschromatographie gereinigt, wobei ca. 4 g Protein/l Gel adsorbiert werden konnten. In einer Säule mit einem Verhältnis Innendurchmesser : Gelbetthöhe = 1 : 1,9 wurde bei einem linearen Fluß von 0,25 cm/min die Proteinfraktion adsorbiert, anschließend durch Waschen mit 20 mM Tris-HCl, 3 M NaCl, pH 7,4, das Inertprotein entfernt und schließlich durch stufenweise Elution die Faktor II enthaltende Fraktion isoliert, die bei fallender Leitfähigkeit bei 1,9 M NaCl vom Gel desorbierte. Die Faktor II enthaltende Fraktion wurde anschließend direkt an Ceramik-Hydroxylapatit®, Fa. Biorad, adsorbiert. Dies erfolgte auf einer Säule mit einem Verhältnis Innendurchmesser : Gelbetthöhe = 1 : 4,8. Die Elution erfolgte bei einem linearen Fluß von 3 cm/min. Durch Elution mit einem Salzgradienten konnte Faktor II von der Säule desorbiert werden. Die Faktor II enthaltenden Fraktionen wurden gesammelt und über Ultra-/Diafiltration über Polysulfonmembranen mit einem cut-off von 30 kD aufkonzentriert bis die Faktor II-Konzentration 50 - 100 E/ml betrug. Eine so hergestellt Faktor II-Präparation wies eine spezifische Aktivität von mindestens 7 E/mg Protein auf. Andere Gerinnungsfaktoren, insbesondere Faktor IX und Faktor VIII, waren nur mehr in Spuren oder gar nicht mehr, wie im Präparat aus Beispiel 2, nachweisbar. Durch Wahl eines geeigneten Diafiltrationspuffers wurde die Faktor II-Präparation in einem pharmazeutisch verträglichen Puffer (z.B. 4 g Trinatriumcitratdihydrat/l, 8 g NaCl/l, pH 7,0) übergeführt.

### BEISPIEL 4 :

### Gewinnung von Faktor Xa (Zwischenprodukt)

Die wie in Beispiel 1 beschrieben hergestellte Faktor X-Fraktion wurde anschließend wie in DE 43 25 872 beschrieben zu Faktor Xab weiterverarbeitet, wobei die so gewonnene hochgereinigte Faktor Xa-Präparation in Gegenwart von 1 g/100 ml Humanalbumin lyophilisiert wurde. Ein solches Präparat war frei von anderen Gerinnungsfaktoren; der enthaltene Faktor Xa wies eine spezifische Aktivität von 120 E/mg Protein vor Zugabe zum Albumin auf.

### BEISPIEL 5:

### Gefriertrocknung von Faktor II (Zwischenprodukt)

Die in Beispiel 3 beschriebene, hochgereinigten Faktor II enthaltende Präparation wurde ohne Zusatz von Stabilisatoren gefriergetrocknet, wobei nach Lyophilisierung mehr als 80 % der Ausgangsaktivität erhalten blieben.

### BEISPIEL 6 :

### Colyophilisierung von Faktor II und Faktor Xa

Eine gemäß Beispiel 3 hergestellte Faktor II-Präparation wurde in einer Konzentration von 100 E/ml zu 20 ml in ein 50 ml-Fläschchen abgefüllt und bei -80°C schockgefroren. Anschließend wurde eine Lösung eines hochgereinigten Faktor Xa, der gemäß DE 43 25 852 hergestellt worden war und eine Konzentration von 500 E/ml aufwies, in einer Menge von 30 µl auf die gefrorene Faktor II-Lösung dosiert. Durch das sofortige Einfrieren des geringen Volumens wurde eine Mischung von Faktor II- und Faktor Xa-Phase verhindert. Anschließend wurde gefriergetrocknet. Zur Bereitung der Infusionslösung wurde das Lyophilisat mit 20 ml A.dest. rekonstituiert, gemischt und sofort zur Verabreichung vorbereitet.

### BEISPIEL 7 :

### Pharmazeutische Formulierung von Faktor II, Faktor Xa und Antithrombin III und/oder Antithrombin III-Heparinkomplex

Der hochgereinigte Faktor II in Kombination mit Faktor Xa und Antithrombin III oder Antithrombin III-Heparinkomplex wurden in einem Puffer, enthaltend 4 g Trinatrium-citratdihydrat/l und 8 g NaCl/l, pH 7,0, auf die Anwendungskonzentration verdünnt. Diese Lösungen konnten gefriergetrocknet werden, wobei eine Aktivität von zumindest 80 % der jeweiligen Komponenten erhalten blieb.

### BEISPIEL 8 :

### Nachweis der partiellen Prothrombinase

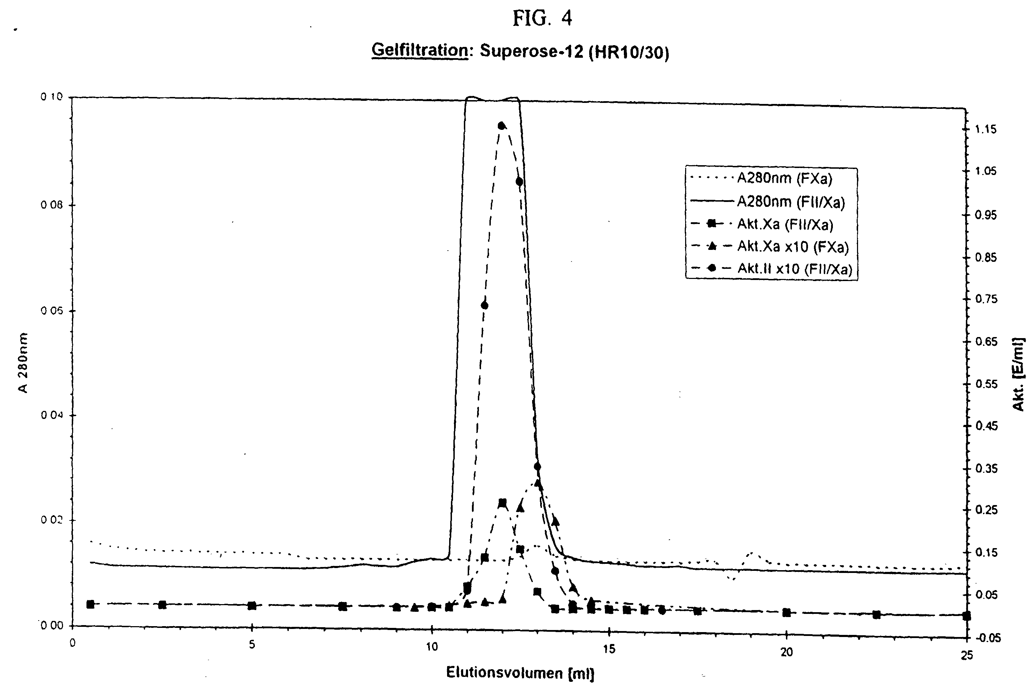

Die Bildung eines Komplexes aus Faktor II und Faktor Xa zur "partiellen Prothrombinase" wurde durch folgendes Experiment nachgewiesen:
57 E Faktor II aus Beispiel 3 und 1,2 E Faktor Xa aus Beispiel 4 wurden in 20 mM Tris-HCl-Puffer, enthaltend 150 mM NaCl, pH 7,4, gelöst und 15 min bei Raumtemperatur zur Bildung des Komplexes inkubiert. Anschließend wurde ein Aliquot der Lösung durch Gelpermeationschromatographie über Superose 12 (HR10/30) (Pharmacia) mit einer Flußrate von 0,25 ml/min chromatographiert. Das Auftragsvolumen betrug 200 µl. Der Durchfluß durch die Säule wurde bei 280 nm UV-spektrophotometrisch gemessen und in Fraktionen zu 0,5 ml gesammelt. Anschließend wurde in den Fraktionen Faktor Xa, der mit der in DE 43 25 872 beschriebenen Methode in einem photometrischen Test mit chromogenem Substrat bestimmt wurde, quantitativ gemessen. Ebenso wurde die Faktor II-Aktivität wie in Beispiel 2 in den einzelnen Fraktionen bestimmt. Das Resultat ist Fig. 4 zu entnehmen. Faktor Xa [Fig. 4: - 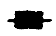 -Akt. Xa (FII/Xa)] und Faktor II [Fig. 4: - 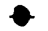 - Akt.II x 10(FII/Xa)] eluierten gemeinsam mit der Proteinfraktion [Fig. 4: ― A280nm (FII/Xa)]. Unter identen Bedingungen wurde nun Faktor Xa alleine auf die Säule aufgetragen, das Elutionsprofil nach der Gelpassage durch Messung der UV-Absorption bei 280 nm bestimmt [Fig. 4: ····· A280nm (FXa)] und Faktor Xa-Aktivität [Fig. 4: - 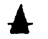 - Akt.Xa x10 (FXa)] in den Fraktionen nachgewiesen. Der dem Faktor Xa entsprechende Protein-Peak lag deutlich abgesetzt vom Faktor Xa im Komplex mit Faktor II. Durch Reduktion der Retentionszeit des Faktor Xa im Komplex auf der Säule und damit verbunden zur Verschiebung zu einer scheinbar höheren Molmasse, konnte der Nachweis einer Komplexbildung von Faktor II und Faktor Xa (partielle Prothrombinase) erbracht werden.

### BEISPIEL 9 :

### Formulierung des Präparates im Zweikammerspritzensystem

Zur Vereinfachung der Anwendung des Mehrkomponentensystemen, wie z.B. Mischungen von Faktor II und Faktor Xa oder Faktor II, Faktor Xa und Antithrombin III, kann als Applikationsvorrichtung ein doppelter Doppelkammerspritzenkörper, wie er in AT 382 783 beschrieben ist, verwendet werden. Bei der klinischen Anwendung von lyophilisierten Mehrkomponentensystemen müßten diese sonst jeweils rekonstituiert werden und in einem definierten Verhältnis miteinander gemischt werden, bevor sie dem Patienten verabreicht werden können. Die Verfüllung der entsprechenden Lyophilisate in einem Doppelkammerspritzensystem ermöglicht eine exakte Dosierung auf eine vorherbestimmbare Aktivität des Präparates zur Behandlung von Inhibitorhaemophilie, z.B. auf konventionelle FEIBA-Einheiten, die gemäß AT 350 726 ermittelt werden können. Unter Verwendung eines solchen Systemes wird die wirksame Mischung in situ bei der Injektion hergestellt. In einer Ausführungsform liegen in den beiden Doppelkammerspritzenkörpern jeweils Lyophilisate der Wirkstoffe, Faktor II und Faktor Xa, vor, die durch Zugabe des Lösungsmittels, A.dest., aufgrund der leichten Löslichkeit der hochgereinigten Proteine sofort in Lösung treten und unmittelbar durch Weiterdrücken der Spritzenkolben nach Mischung im Mischkopf dem Patienten infundiert werden (siehe Fig. 1).

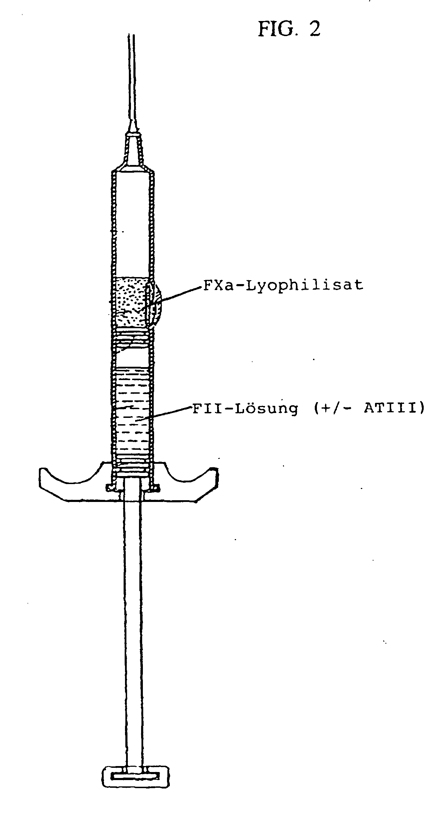

Aufgrund der hohen Stabilität des Faktor II in Lösung, eignet sich diese auch als Lösungsmittel für den Faktor Xa in der Darreichungsform einer Doppelkammerspritze. Eine Lösung von hochgereinigtem Faktor II, z.B. hergestellt gemäß Beispiel 2, in einem physiologisch verträglichen Citratpuffer (4 g Trinatriumcitratdihydrat/l, 8 g NaCl/l, pH 7,0) in einer Konzentration von 100 E Faktor II/ml wird mit Antithrombin III, Immuno Wien, versetzt (1 mE Antithrombin III/E Faktor II). In einer Doppelkammerspritze (siehe Fig. 2) wird diese Lösung als Lösungsmittel für das gefriergetrocknete Pulver eines hochgereinigten Faktor Xa verwendet.

### BEISPIEL 10 :

### Stabilität des hochgereinigten Faktor II

Faktor II wurde wie in Beispiel 2 beschrieben gereinigt und als Lösung in einer Konzentration von 60 E/ml in einem Puffer, enthaltend 4 g/l Trinatriumcitratdihydrat, 8 g/l NaCl, pH 7,0, bei 5°C, bei 22°C, bei 37°C und bei 50°C gelagert. Bei den Lagerungstemperaturen 5°C und 22°C wurden Proben jeweils alle 24 h gezogen, bei der Lagerung bei 37°C und bei 50°C erfolgten die Probenentnahmen über 24 h zum Zeitpunkt 1 h, 2 h, 4 h, 8 h und 24 h. In den Proben wurde jeweils Faktor II-Aktivität bestimmt.

Bei 5°C konnten auch noch 86 Tage nach Lagerungsbeginn mehr als 80 % der Ausgangsaktivität festgestellt werden. Bei 22°C wurde über 14 Tage mehr als 80 % der Ausgangsaktivität gefunden; bei 37°C konnten 24 h nach Lagerungsbeginn 95 % der Ausgangsaktivität wiedergefunden werden. Sogar bei 50°C wurden 8 h nach Beginn der Lagerung noch 94 % der ursprünglichen Aktivität gefunden.

### BEISPIEL 11 :

### Autoaktivierung und Stabilität der erfindungsgemäßen Präparate

Präparate gemäß Beispiel 5 (Faktor II) und Beispiel 6 (Faktor II/Xa-Komplex) wurden im Vergleich zu zwei handelsüblichen Prothrombinkomplexkonzentraten in einem in vitro-Test auf ihre prothrombotischen Eigenschaften insbesondere des extrinsischen Gerinnungssystems untersucht. Vor Einsatz im Test wurde das im Prothrombinkomplexkonzentrat vorhandene Heparin entsprechend der Konzentration mit Protaminsulfat neutralisiert, um thrombogene Bestandteile, die durch das Heparin maskiert würden, darzustellen.

Der Analysenansatz bestand aus 250 µl Thrombotest® (Nycomed Pharma AS, Oslo, Norwegen), einem Präparat enthaltend Thromboplastin aus Rinderhirn und adsorbiertem Rinderplasma, welches 3 min bei 37°C vorinkubiert wurde. Anschließend wurden 50 µl einer Probe zugesetzt und die Gerinnungszeit mit einem Kugelkoagulometer (KC4, Amelung) bestimmt. Rinderthromboplastine gelten als besonders sensitiv für aktivierte Gerinnungsfaktoren. Entsprechend kann das Testsystem eine Aussage über die in vitro-Thrombogenität der untersuchten Präparate geben. In diesem Testansatz wurde humanes Normalplasma als Kontrolle unverdünnt eingesetzt. Dieses zeigt eine Gerinnungszeit von 74 sek. Die beiden untersuchten erfindungsgemäßen Präparate zeigten unverdünnt eingesetzt Gerinnungszeiten von mehr als 100 sek, wobei die Konzentration einer möglichen Anwendungskonzentration von 30 E Faktor II/ml entsprach. Ein handelsübliches Prothrombinkomplexkonzentrat, ebenfalls gelöst in der Anwendungskonzentration und verdünnt auf 30 E/ml, mußte mit Puffer 1 : 32 weiterverdünnt werden, um auf die Gerinnungszeit des Normalplasmas (74 sek) zu kommen. Ein anderes handelsübliches, aktiviertes Prothrombinkomplexkonzentrat mußte sogar 1 : 216 mit Puffer verdünnt werden, um auf die Gerinnungszeit eines Normalplasmas von 74 sek zu kommen. Anschließend wurden die erfindungsgemäßen Präparate bis zu 4 h bei Raumtemperatur gelöst gelagert und ein mal pro Stunde Proben gezogen und neuerlich die Gerinnungszeit mit Thrombotest® bestimmt. Dabei kam es zu keiner Veränderung, d.h. auch nach 4 h zeigten die unverdünnt eingesetzten Proben immer noch Gerinnungszeiten über 100 sek. Aus diesen Versuchen war zu ersehen, daß die erfindungsgemäßen Präparate gegenüber handelsüblichen Prothrombinkomplexkonzentraten ein deutlich geringeres Thrombogenitätspotential aufweisen.

### BEISPIEL 12 :

### Lösungsverhalten des Präparates

Eine hochgereinigten Faktor II enthaltende Präparation wurde, wie in Beispiel 5 beschrieben, hergestellt und gefriergetrocknet. Das Lyophilisat war so eingestellt, daß die Volumsaktivität nach Rekonstitution mit destilliertem Wasser 50 E/ml war. Dies war eine typische Anwendungskonzentration. Aufgrund der hohen Reinheit des Präparates konnten jedoch auch Volumskonzentrationen von 100 und mehr Einheiten Faktor II pro ml erzielt werden. Die Lösezeit des Präparates, definiert als jene Zeit die von der Zugabe des Lösungsmittels (destilliertes Wasser) bis zum vollständigen Auflösen des Pulvers, wurde bestimmt. Im Vergleich dazu wurden die Lyophilisate zweier handelsüblicher Präparate, nämlich Prothrombinkomplex und aktivierter Prothrombinkomplex, nach Angabe des Herstellers jeweils in der Anwendungskonzentration durch Zugabe von destilliertem Wasser rekonstituiert und ebenso die Lösezeit bestimmt. Die Lösezeit der untersuchten Präparate ist der nachfolgenden Tabelle zu entnehmen:

| | **Präparat gemäß Beispiel 5** | **APCC**^{**1**} | **PCC**^{**2**} |
|---|---|---|---|
| **Lösezeit (s)** | < 30 | 240 | 300 |

| | | | |
|---|---|---|---|
| ¹APCC = aktiviertes Prothrombinkomplexkonzentrat | | | |
| ²PCC = Prothrombinkomplexkonzentrat | | | |

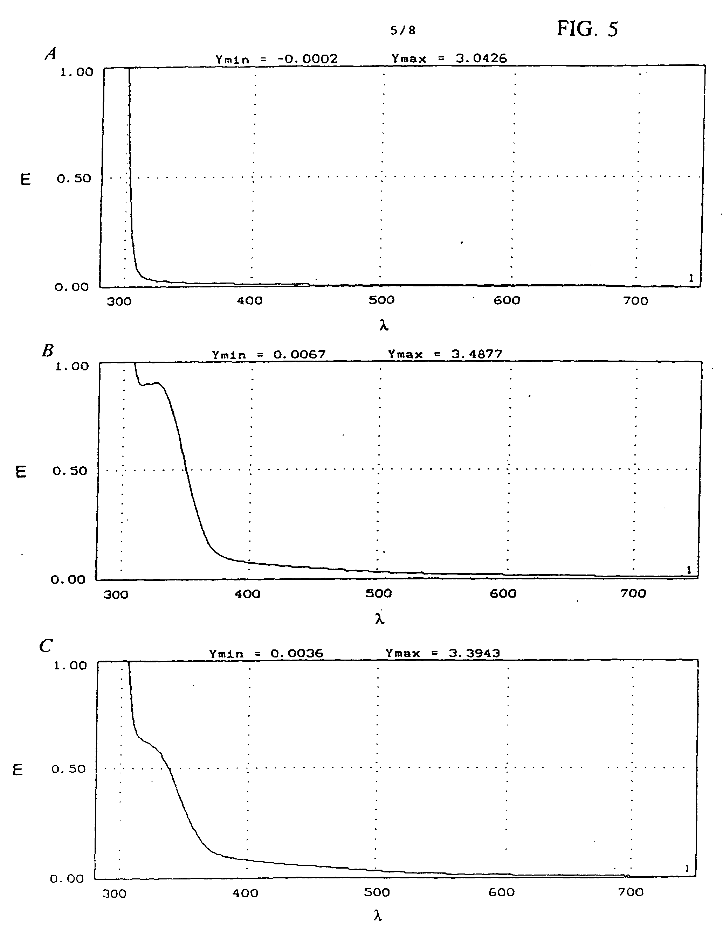

Nach erfolgter Lösung der jeweiligen Präparate wurde von diesen jeweils ein UV/VIS-Spektrum zwischen 280 und 750 nm gegen einen Citrat-Kochsalzpuffer gemessen. Die Spektren sind Fig. 5a, b und c zu entnehmen (a: Präparat gemäß Beispiel 5, b: aktivierter Prothrombinkomplex, c: Prothrombinkomplex). Der Vergleich der Spektren zeigte, daß das Präparat gemäß Beispiel 5 im sichtbaren Bereich bis 700 nm eine deutlich niedrigere Lichtabsorption aufwies als die Vergleichspräparate. Das Präparat gemäß Beispiel 5 zeichnete sich also durch eine hervorragende Löslichkeit aus und war in gelöster Form als klare, ungefärbte Lösung, wie für ein verbessertes pharmazeutisches Präparat charakteristisch, vorliegend.

### BEISPIEL 13 :

### Wirkung des Präparates im Faktor VIII-Inhibitorplasma

Ein hochtitriges Faktor VIII-Inhibitorplasma (55 BU/ml) wurde im Eisbad vorgekühlt und mit PTT-Reagens (IMMUNO, Wien) und der zu testenden Probe, beide ebenfalls im Eisbad vorgekühlt, in einem Verhältnis von 1 + 1 + 1 bei 37°C 1 min inkubiert. Anschließend wurde 1 Teil einer 0,05 M CaCl₂-Lösung zugesetzt und die Gerinnungszeit des Testansatzes mit einem Kugelkoagulometer der Fa. Amelung, Modell KC-4, bestimmt. Die zu testenden Präparationen wurden in einem Puffer, enthaltend 7 g NaCl/l und 6 g Trinatriumcitratdihydrat/l, 1 : 10 weiterverdünnt und mit dieser Konzentration im Test eingesetzt. Unter diesen Bedingungen wurde hochgereinigter Faktor II, alleine und in Kombination mit hochgereinigtem Faktor Xa, in Kombination mit Antithrombin III und Heparin getestet. Die Gerinnungszeiten sind nachfolgender Tabelle zu entnehmen.

| Testsubstanzen (Konzentration) | | | | Gerinnungszeit (sek) |
|---|---|---|---|---|
| Faktor II | Faktor Xa | Antithrombin III | Heparin | |
| 10 E/ml | - | - | - | 147 |
| 10 E/ml | 0,1 E/ml | - | - | 63 |
| 10 E/ml | 0,1 E/ml | 1 E/ml | - | 55 |
| 10 E/ml | 0,1 E/ml | 1 E/ml | 6 E/ml | 49 |

Als Kontrolle wurde die Gerinnungszeit des Testansatzes mit reinem Natriumchlorid-Trinatriumcitrat-Puffer bestimmt. Diese betrug 148 sek. Somit konnte nur durch den Zusatz von Faktor II/Xa mit oder ohne Antithrombin III oder Heparin die Gerinnungszeit des Inhibitorplasmas deutlich reduziert werden. Durch Messung einer FEIBA-Standardpräparation (FEIBA STIM 4, Immuno), welche das konventionelle Präparat zur Behandlung von Faktor VIII-Inhibitorpatienten darstellt, in verschiedenen Konzentrationen im selben Testansatz konnte ermittelt werden, daß die FEIB-Aktivität des Präparates, enthaltend 10 E/ml Faktor II und 0,1 E Faktor Xa/ml, ca. 25 E FEIBA/ml entspricht.

### BEISPIEL 14 :

### In vivo-Wirkung bei Faktor VIII-Inhibitorhaemophilie (Vergleichsversuch)

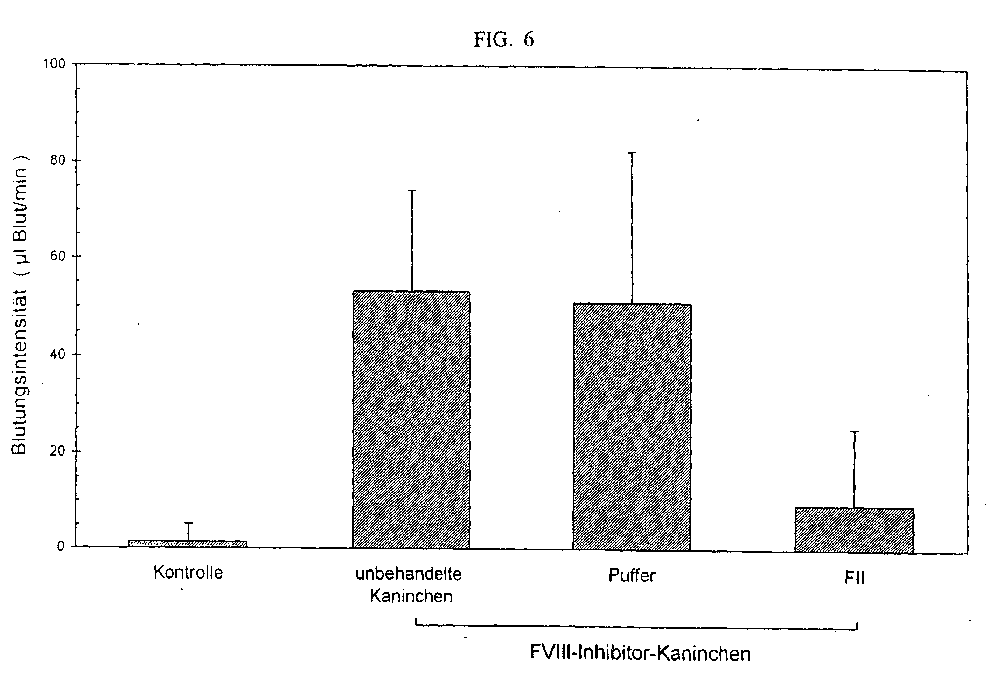

Zur Testung der in vivo-Wirksamkeit des erfindungsgemäßen Präparates wurde ein Faktor VIII-Inhibitorhaemophilie Kaninchenmodell verwendet. Ca. 2 kg schwere, weiße Neuseelandkaninchen wurden narkotisiert. Nach Eintritt der Narkose wurde jeweils die rechte Vena femoralis präpariert und ein permanenter venöser Zugang geschaffen. Durch diesen wurden 0,5 ml/kg Körpergewicht eines human Faktor VIII-Inhibitorplasmas (1500 BU/ml) über 10 min infundiert. 30 min nach Abschluß der Infusion wurde die Blutungscharakteristik unter Verwendung einer modifizierten Methode nach Giles et al., Blood 60:727-730 (1982), bestimmt. Dazu wurde das Fell um eine Kralle der Hinterpfote des Kaninchens rasiert, um zu verhindern, daß bei der späteren Blutung austretendes Blut vom Fell absorbiert wird. Die Nagelhaut wurde mittels einer Krallenzange verletzt; unmittelbar danach wurden Filter darunter so etabliert, daß das Blut direkt auf den Filter tropfen konnte, ohne von diesem durch Kapillarwirkung aufgesogen zu werden, um zu verhindern, daß ein sich formierendes Blutgerinnsel zerstört würde. Die Filtereinheiten wurden alle 2 min gewechselt, das austretende Blut in Fraktionen gesammelt. Die Blutsammlung wurde 30 min fortgesetzt, danach wurde, sofern die Blutung nicht zum Stillstand gekommen war, die Wunde verödet. Zur Quantifizierung der Blutungscharakteristik wurden die Filter mit jeweils 0,04 %iger Ammoniumhydroxidlösung über 5 h extrahiert, dabei lysierten die Erythrozyten, die mit dem Blut im Filter gesammelt wurden. Durch eine 10-minütige Ultraschallbehandlung wurde das Haemoglobin extrahiert und quantitativ, photometrisch bei 416 nm gegen eine Eichkurve bestimmt. Diese wurde so erstellt, indem Kaninchenblut in Volumina zwischen 10 µl und 1 ml auf Filter pipettiert wurden, diese wie oben extrahiert und das Haemoglobin photometrisch bei 416 nm bestimmt wurde. Die Blutungscharakteristik des Nagelschnittes wurde ermittelt, indem graphisch die Blutmengen pro 2 Minuten-Fraktion gegen die Zeit aufgetragen wurden. Zur Beurteilung wurde der kumulierte Blutverlust ermittelt, indem das Volumen der einzelnen Blutfraktionen graphisch gegen die Zeit eingetragen wurde. Als für die Blutung relevantes Kriterium wurde die Steigung der kumulierten Blutung zwischen 10 und 20 min herangezogen. Dieser Wert war unabhängig von der initialen Blutmenge, die von Kaninchen zu Kaninchen je nach Krallenschnitt Schwankungen unterworfen war. Die Steigung der Blutungscharakteristik in 10 bis 20 min Beobachtungsintervallen diente als Maß für die Intensität der Blutung. Eine Steigung gleich Null bedeutete, daß die Blutung zum Stillstand gekommen war, eine Steigung >0 mit einem Korrelationskoeffizienten von > 0,8, daß eine konstante Blutung vorlag. Gesunde Kaninchen wiesen unter den Testbedingungen eine Blutungsintensität von <2 µl Blut/min auf. Faktor VIII-Inhibitorkaninchen zeigten eine Blutungsintensität von ca. 50 µl Blut/min (siehe Fig. 6). Faktor VIII-Inhibitorkaninchen, die mit einer Faktor II-Präparation gemäß Beispiel 3 in einer Dosierung von 75 E/kg Körpergewicht als Infusion über 30 Minuten behandelt wurden, zeigten im Mittel (n = 6) eine Blutungsintensität von 9,3 µl/min und somit eine signifikante Reduktion gegenüber dem unbehandelten Inhibitortier.

### BEISPIEL 15 :

### In vivo-Wirkung bei von Willebrand Faktor/Faktor VIII-Inhibitor

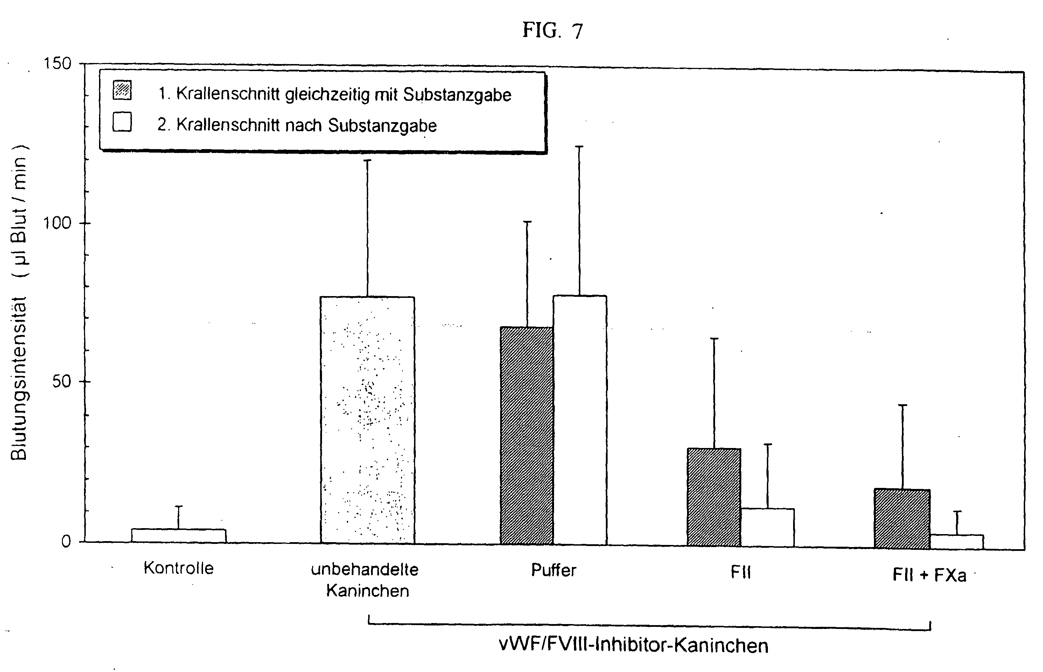

Analog zu Beispiel 14 wurde ein von Willebrand Faktor/Faktor VIII-Inhibitormodell etabliert, indem Kaninchen ein anti-von Willebrand Faktor/Faktor VIII-Antiplasma aus der Ziege, welches durch Immunisierung von Ziegen mit einer gereinigten Faktor VIII/von Willebrand Faktor-Präparation gewonnen wurde, in einer Dosierung von 1 ml/kg Körpergewicht infundiert wurde. Derart vorbehandelte Tiere zeigten eine gesteigerte Blutungsneigung (siehe Fig. 7). Die Blutungscharakteristik wurde durch Krallenschnitte bei diesen Tieren gleichzeitig mit Infusion der Testsubstanz und 30 min nach Abschluß der Infusion der Testsubstanz gemessen. Durch Gabe von Faktor II als Bolus von 2,5 ml in einer Dosierung von 75 E/kg konnte die initial gesteigerte Blutungsintensität von 77 µl/min auf 31 µl/min (1. Krallenschnitt) bzw. 12 µl/min (2. Krallenschnitt) reduziert werden. Durch Kombination von Faktor II (75 E/kg) mit einem Faktor Xab (hergestellt gemäß Beispiel 4) in einer Dosierung von 0,55 E/kg als Bolus mit einem Injektionsvolumen von 2,5 ml konnte die Blutungsneigung vom Ausgangswert auf 18 µl/min beim 1. Krallenschnitt und 5 µl/min beim 2. Krallenschnitt reduziert werden. Damit wurde das abnorm gesteigerte Blutungsverhalten der Inhibitortiere auf das Niveau der gesunden Vergleichstiere normalisiert (4 µl/min).

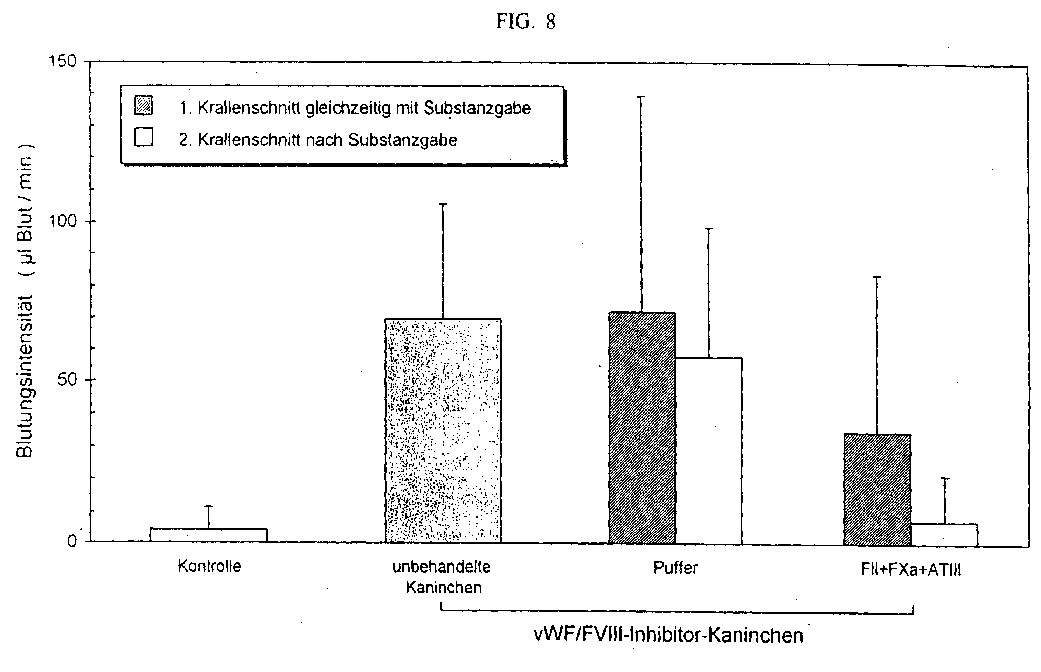

Zusätzlich wurde auch die Kombination von Faktor II (75 E/kg), Faktor Xa (0,55 E/kg) und Antithrombin III, Immuno Wien, (75 mE/kg) im selben Modell untersucht. Dabei konnte beim 1. Krallenschnitt eine Reduktion auf 35 µl/min und beim 2. Krallenschnitt auf 7 µl/min erreicht werden (siehe Fig. 8). Der Zusatz von Antithrombin III sollte eine Generierung von Thrombin aus Prothrombin durch das Enzym Faktor Xa in der verabreichten Lösung verhindern.

### BEISPIEL 16 :

### Thrombogenität des erfindungsgemäßen Präparates

Die Einzelkomponenten und Mischungen derselben des erfindungsgemäßen Präparates wurden auf ihre thrombogene Aktivität unter Verwendung der von Wessler beschriebenen Methode, J.Appl.Phys. 14:943-946 (1959), im stasierenden venösen Blut in Kaninchen getestet.

Kaninchen wurden in Pentobarbital-Narkose versetzt, die Vena jugularis der Tiere wurde freipräpariert und mit losen Ligaturen im Abstand von 1 - 2 cm versehen. Die zu testenden Substanzen wurden den Tieren in die der freipräparierten Vena jugularis gegenüberliegende Ohrvene injiziert. Die Injektion erfolgte innerhalb von 15 sek. Nach einer Wartezeit von 10 - 15 sek wurde das Venenstück abgeklemmt. Nach weiteren 10 min wurde das abgeklemmte Venenstück entnommen und in einem Citratpuffer in einer Petrischale aufgeschnitten und die erhaltenen Thromben mittels einer Skalierung zwischen 0 und 4 bewertet (siehe Tabelle).

| **THROMBOSEGRAD** | **BEWERTUNG** |
|---|---|
| keine Thrombendildung | 0 |
| wenige kleine Thromben | 0,5 - 1 |
| wenige mittelgroße und viele kleine Thromben | 2 |
| viele mittelgroße Thromben | 3 |
| wenige große Thromben | 3,5 |
| ein zusammenhängender Thrombus | 4 |

Die Substanzen wurden an jeweils sechs Tieren untersucht, die je 75 E Faktor II/kg, 0,55 E Faktor Xa/kg und 75 mE Antithrombin III/kg entweder alleine oder in Kombination erhielten. Die nachfolgende Tabelle gibt den Mittelwert des Wessler-Scores von jeweils sechs untersuchten Tiere wieder. Als Kontrolle wurde reiner Citratpuffer, der auch als Verdünnungspuffer verwendet wurde, herangezogen.

| **Testsubstanz** | | | |
|---|---|---|---|
| **Faktor II** | **Faktor Xa** | **Antithrombin III** | **Wessler Score** |
| + | - | - | 0,17 |
| - | + | - | 0,17 |
| - | - | + | 0,08 |
| + | + | - | 0,25 |
| + | - | + | 0,08 |
| - | + | + | 0,17 |
| + | + | + | 0,17 |
| - (Puffer) | - (Puffer) | - (Puffer) | 0,2 |

Es zeigte sich, daß keine der eingesetzten Komponenten, weder alleine noch in Kombinationen, eine thrombogene Aktivität im Kaninchen aufwies.

### BEISPIEL 17 :

### Wirkung des erfindungsgemäßen Präparates in Abhängigkeit der Darreichungsform

Eine Faktor II und Faktor Xa enthaltende Präparation gemäß Beispiel 6 wurde in jeweils 6 Kaninchen mit induzierter Faktor VIII-Inhibitorhaemophilie (siehe Beispiel 14) auf seine Wirksamkeit getestet. Die untersuchte Dosis war wie in Beispiel 15 beschrieben. Als Kontrolle wurde den Tieren reiner Puffer infundiert.

Im ersten Versuch wurde eine Infusion der Testsubstanzen über 30 min, entsprechend ca. 15 min/kg Körpergewicht, mittels einer automatischen Infusionspumpe, bei einer Infusionsgeschwindigkeit von 1 ml/min durchgeführt. In einem zweiten Versuch wurde die selbe Dosis, jedoch als Bolus innerhalb von 30 sek in einem kleinen Injektionsvolumen von 2,5 ml/kg Körpergewicht den Tieren gegeben. Wie in Beispiel 14 wurde nun die Blutungsintensität während und nach erfolgter Substanzgabe gemessen. Es zeigte sich, daß sowohl bei langsamer Infusion mit einem großen Infusionsvolumen, als auch bei der schnellen Injektion einer kleinen Volumsdosis, aber mit identen Dosen bezogen auf das Körpergewicht, die pathologisch verlängerte Blutungsintensität von 67 µl/min auf ca. 5 µl/min, entsprechend dem Blutungsverhalten der gesunden Vergleichstiere, normalisiert werden konnte. Durch Gabe von Puffer, ebenso mit beiden Infusionsmodalitäten, wurde keine Änderung der Blutungsintensität gefunden. Trotz der schnellen Injektion als Bolus wurde keine Unverträglichkeitsreaktion bei der Injektion, wie auch bei der langsamen Infusion beobachtet. Für handelsübliche Präparate mit vergleichbarer Indikation (aktivierte) Prothrombinkomplexpräparate werden Infusionsgeschwindigkeiten im Bereich von < 0,05 ml/min/kg Körpergewicht empfohlen, um akute thromboemolische Nebenwirkungen und Unverträglichkeitsreaktionen zu vermeiden. Wie der Versuch zeigte, konnte das erfindungsgemäße Präparat aufgrund seiner hohen Reinheit mit 5 ml/min/kg Körpergewicht, also mit der 100-fachen Injektionsgeschwindigkeit nebenwirkungsfrei verabreicht werden.

### BEISPIEL 18 :

### Wirkung von partieller Prothrombinase in Ratten mit Plättchendefekt

Als Thrombozytopathie-Modell werden Haubenratten, die von T.B. Tschopp und M.B. Zucker (Hereditary Defect in Platelet Function in Rats, Blood 1972; 40: 217-226) beschrieben wurden, verwendet. Dieser Rattenstamm, der von S.L. Raymond und W.J. Dodds (Characterization of the Fawn-Hooded Rats as a Model for Hemostatic Studies, Thrombos Diathes haemorrh. 1975; 33: 361-369) weiter charakterisiert wurde, zeichnet sich durch abnorm gesteigerte Blutungszeit und reduzierte Plättchenaggregation aus, während Prothrombinzeit, partielle Thromboplastinzeit, Plasma Faktor VIII- und Fibrinogenspiegel sowie Plättchenzahl im gleichen Bereich wie in der gesunden Ratte liegen. Die in der Haubenratte auftretende Thrombopathie ist mit einer reduzierten Thrombininduzierten ATP- und ADP-Freisetzung sowie mit einer reduzierten Serotoninfreisetzung verbunden, und wird daher als "Storage-Pool Defizienz" charakterisiert, die "Storage-Pool Defizienz" beim Menschen widerspiegeln.

Die Haubenratten werden mit Ketamin-Xylazin (100 mg/kg + 5 mg/kg) i.m. narkotisiert. In eine Jugularvene wird ein Katheter eingebunden, über welchen die zu testenden Substanzen infundiert werden. Anschließend wird die Blutungszeit bestimmt, wobei hierzu Simplate R-Einmalschnäpper (Organon, Technica) verwendet werden. Im Abstand von ca. 1,5 cm von der Schwanzwurzel werden dorsal und danach ventral je ein Längsschnitt angebracht und jeweils die Blutungszeit gemessen. Der Mittelwert aus den beiden Einzelmessungen gilt als Blutungszeit. Danach erfolgt die Applikation der Testsubstanz (Puffer, Faktor II gemäß Beispiel 2, Faktor Xa gemäß Beispiel 4 und partielle Prothrombinase gemäß Beispiel 6); 30 Minuten später wird die Blutungszeit neuerlich bestimmt. Zur Kontrolle werden gesunde Sprague-Dawley Ratten (Charles River) verwendet.

Die Untersuchung erfolgt in Gruppen von je 10 Tieren, wobei der Komplex der partiellen Prothrombinase in 2 Dosen, 75 E/kg Körpergewicht (KG) Prothrombin und 0,55 E/kg KG Faktor Xa sowie 150 E/kg KG Prothrombin und 1,1 E/kg KG Faktor Xa, verabreicht wird. Als Kontrolle dazu werden die Einzelkomponenten und Puffer gegeben. Die in den einzelnen Versuchsgruppen gemessenen mittleren Blutungszeiten sind in der nachfolgenden Tabelle angegeben. Gesunde Ratten weisen unter den gleichen Versuchsbedingungen eine Blutungszeit von 168 ± 5 Sekunden auf, während Ratten mit einer Thrombopathie eine verlängerte Blutungszeit von 335 ± 10 Sekunden haben. Durch Gabe der partiellen Prothrombinase kann diese abnorm gesteigerte Blutungszeit auf ca. 270 Sekunden reduziert werden.

**Tabelle**

| **Blutungszeit (sek)** | | **Faktor II (E/kg)** | | |
|---|---|---|---|---|
| **Mittelwert aus 10 Tieren** | | **0** | **75** | **150** |
| | 0 | 335 ± 10 | 296 ± 13^{ns} | 307 ± 11^{ns} |
| **FXa (E/kg)** | 0,55 | n.b. | 273 ± 13* | n.b. |
| | 1,1 | 320 ± 13^{ns} | n.b. | 268 ± 11*** |
| **Statistische Auswertung:** Die Daten sind als Mittelwerte ± Standardabweichung angegeben. Die Gruppenmittelwerte wurden mittels Student T-Test verglichen. n.b. nicht bestimmt | | | | |

| | | | | |
|---|---|---|---|---|
| ^{ns} Unterschied zur Kontrollgruppe (0 E/kg FII und 0 E/kg FXa) nicht signifikant | | | | |
| *Unterschied zur Kontrollgruppe (0 E/kg FII und 0 E/kg FXa) signifikant mit p ≥95 % | | | | |
| ***Unterschied zur Kontrollgruppe (0 E/kg FII und 0 E/kg FXa) signifikant mit p ≥ 99,9 % | | | | |

### BEISPIEL 19 :

### Wirkung von partieller Prothrombinase im von Willebrand Faktor-defizienten Hund

Ein Hund mit angeborener von Willebrand Faktor-Defizienz mit einem nicht meßbaren von Willebrand Faktor-Aktivitäts- und Antigenplasmaspiegel und einer um 50 % verminderten Faktor VIII-Plasmakonzentration wird mit einer partiellen Prothrombinase in einer Dosis von 100 E/kg KG behandelt. Dazu wird der Hund narkotisiert'und anschließend die partielle Prothrombinase hergestellt gemäß Beispiel 6 als Bolus intravenös verabreicht. Unmittelbar vor Verabreichung der Testsubstanz sowie 15 min, 30 min, 1 h, 2 h, 3 h, 24 h und 48 h nach Infusion werden Blutproben genommen, aus diesen Plasma hergestellt und die Prothrombin-, Thrombin- und Faktor VIII-Konzentration in den Plasmaproben bestimmt.

Vor Verabreichung der partiellen Prothrombinase sowie 3 und 24 h nach der Injektion wird die Nagelhautblutungscharakteristk bestimmt. Dazu wird nach der Methode von A.R. Giles, S. Tinlin und R. Greenwood, A Canine Model of Hemophilic (Factor VIII:C Deficiency) Bleeding, Blood 1982; 60, in abgewandelter Form vorgegangen. Das Fell um die Kralle wird rasiert, um zu verhindern, daß bei der späteren Blutung austretendes Blut vom Fell absorbiert wird. Die Nagelhaut wird mittels einer Krallenzange verletzt. Unmittelbar danach werden Filter (Pipetman P5000-Schutzfilter, Gilson) unter der Wunde so etabliert, daß das Blut direkt auf den Filter tropfen kann, ohne von diesem durch Kapillarwirkung aufgesogen zu werden, um zu verhindern, daß ein sich formierendes Blutgerinnsel verletzt wird. Die Filtereinheiten werden alle 2 Minuten gewechselt und das austretende Blut in Fraktionen gesammelt. Die Blutsammlung wird 30 min fortgesetzt. Danach wird, sofern die Blutung nicht zum Stillstand gekommen ist, die Wunde verödet. Verschiedene Krallen können bei ein und demselben Tier verwendet werden.

Die Qualifizierung der Blutungscharakteristik erfolgt durch Extraktion des in Fraktionen auf den Filtern gesammelten Blutes mit jeweils 5 ml 0,04 % Ammoniumhydroxid-Lösung über 5 Stunden. Dabei lysieren die Erythrozyten, die mit dem Blut im Filter gesammelt wurden. Durch eine 10-minütige Ultraschallbehandlung (Sonorex RK 100, Bandelin electronic, Berlin) wird das Haemoglobin extrahiert und quantitativ photometrisch bei 416 nm gegen eine Eichkurve bestimmt. Eine Eichkurve kann ermittelt werden, indem Hundeblutvolumina zwischen 10 µl und 1 ml auf die Filter pipettiert werden, diese wie oben beschrieben extrahiert werden und das Haemoglobin photometrisch bei 416 nm bestimmt wird. Entsprechend lassen sich lineare Eichkurven erstellen, die eine direkte Umrechnung der Haemoglobinkonzentration auf die Blutmenge pro Filter ermöglichen. Die Blutungscharakteristik des Nagelschnittes wird ermittelt, indem graphisch die Blutmenge pro 2-Minuten-Fraktion gegen die Zeit aufgetragen wird. Zur Beurteilung der Blutungscharakteristik wird der kumulierte Blutverlust ermittelt, indem die einzelnen Blutfraktionen additiv gegen die Zeit in die Graphik eingetragen werden. Als für die Blutung relevantes Kriterium wird die Steigung der kumulierten Blutung zwischen 10 und 20 Minuten herangezogen. Dieser Wert ist unabhängig von der initialen Blutmenge, die durch schlecht standarisierbare Krallenschnittechniken variieren kann. Die Steigung dieser Blutungscharakteristik im 10 - 20 min Beobachtungsintervall dient als Maß für die Intensität der Blutung und wird in ml Blut/min angegeben. Eine Steigung gleich Null, entsprechend 0 ml/min, bedeutet, daß die Blutung zum Stillstand gekommen ist; eine Steigung größer als Null mit einem Korrelationskoeffizienten von > 0,8 bedeutet, daß eine konstante Blutung vorliegt.

Normale Hunde, die unter diesen Bedingungen getestet wurden zeigen im Beobachtungszeitraum keine Blutung mehr, d.h. die Blutung ist bereits zuvor zum Stillstand gekommen (Blutungsintensität: 0,0 ml/min).

Die Blutungsintensität betrug vor Verabreichung der partiellen Prothrombinase 1,05 ml/min und war nach 3 h auf 0,35 ml/min und auch nach 24 h weiterhin auf 0,47 ml/min reduziert.

Der Faktor VIII-Plasmaspiegel blieb über die gesamte Beobachtungszeit konstant, von Willebrand Faktor-Antigen blieb unter der Nachweisgrenze, Prothrombin war vor Substanzgabe bei 0,7 E/ml und stieg nach Injektion der partiellen Prothrombinase auf 2,4 E/ml an und wurde mit einer Halbwertszeit von ca. 24 h aus der Zirkulation eliminiert. 1 h nach Verabreichung der Testsubstanz konnte eine signifikante Thrombingenerierung mit einem chromogenen Substrat für Thrombin, Th1 (Immuno), bestimmt werden. Die Injektion von partieller Prothrombinase wurde ohne Nebenwirkungen vom Hund vertragen.

### BEISPIEL 20 :

### Wirkung von partieller Prothrombinase und von Willebrand Faktor im von Willebrand Faktor-defizienten Hund

Analog zu Beispiel 19 wurde der Hund mit einer partiellen Prothrombinase in einer Dosierung von 100 E/kg KG und einem gereinigten von Willebrand Faktor in einer Dosierung von 60 RCoF E/kg KG behandelt. Durch Gabe dieser Kombination kam es im von Willebrand Faktor-defizienten Hund nach 24 h zu einer vollständigen Normalisierung des abnorm gesteigerten Blutungsverhaltens, wobei die Plasmaparameter wie in Beispiel 19 bezüglich Prothrombin und Thrombin vergleichbar waren. Darüberhinaus kam es zu einem endogenen Faktor VIII-Anstieg auf ca. 200 % des Ausgangswertes der über einen Zeitraum von mehr als 48 h konstant blieb und anschließend über einen Zeitraum von ca. 120 h zum Ausgangswert zurückkehrte. Von Willebrand Faktor wurde mit einer Halbwertszeit von 20 h eliminiert. Aus der Literatur (L. Drouet, J. Roussi, M. Bonneau, G.A. Pignaud, P.L. Turecek, F. Dorner, U. Schlokat, F.G. Falkner, B. Fischer, A. Mitterer und H.P. Schwarz, The effect of recombinant human von Willebrand Factor in pigs with severe von Willebrand Disease. Blood 1995; 86: 612a-AbsNo2435. (abs)) war bekannt, daß die Gabe von von Willebrand Faktor alleine lediglich zu einer partiellen Normalisierung des Blutungsverhaltens führt.

### BEISPIEL 21 :

### Wirkung der partieller Prothrombinase als Antagonist von Peptidantikoagulantien

Eine partielle Prothrombinase in einer Zusammensetzung von 57 E Faktor II und 1,2 E Faktor Xa wurde in 20 mM Tris-HCl-Puffer, enthaltend 150 mM NaCl, pH 7,4, gelöst und 15 min bei Raumtemperatur zur Bildung des Komplexes inkubiert. Anschließend wurde ein Aliquot von 50 µl entnommen und mit 50 µl eines Tris-Imidazolpuffers, pH 8,4, für 90 sek bei 37°C inkubiert. Anschließend wurde mit 100 µl einer Lösung des chromogenen Substrates, Methoxycarbonyl-D-cyclohexylalanyl-glycyl-L-arginin-p-nitroanilidhydroacetat, versetzt, so daß die Konzentration des chromogenen Substrates 1 mmol/l betrug. Anschließend wurde die Kinetik der Spaltung des chromogenen Substrates photometrisch bei 405 nm über 3 min bei 37°C bestimmt. Das chromogene Substrat, welches sowohl von Thrombin als auch von Faktor Xa hydrolysiert wird, wies bei Inkubation mit partieller Prothrombinase eine ΔOD/min von 0,084 auf. Als Vergleich wurde Faktor Xa in derselben Konzentration jedoch ohne Prothrombin in diesem Test eingesetzt und es zeigte sich eine Umsatzrate des chromogenen Substrates von 0,064 ΔOD/min. Zur partiellen Prothrombinase wurde rekombinantes Hirudin (Rhein-Biotech) in einer Konzentration von 0,0025 E/ml zugesetzt und ebenso die Umsatzrate des chromogenen Substrates untersucht. Es zeigte sich, daß nach Abzug des Substratumsatzes des reinen Faktor Xa (0,064 ΔOD/min) kein weiterer Substratumsatz mehr gemessen werden konnte, was auf eine vollständige Neutralisierung des Hirudins zurückzuführen war.

Neben dem effektiven und spezifischen Thrombininhibitor, Hirudin, wurde dann auch der selektive Faktor Xa-Inhibitor, rekombinantes Tick-Anticoagulant Peptide, ein rekombinantes Äquivalent des Serinproteaseinhibitors aus Ornithodoros moubata, dessen antikoagulatorische in vivo-Wirksamkeit von G.P. Vlasuk, D.Ramjit, T. Fujita et al. in Comparison of the In Vivo Anticoagulant Properties of Standard Heparin and the Highly Selective Factor Xa Inhibitors Antistasin and Tick Anticoagulant Peptide (TAP) in a Rabbit Model of Venous Thrombosis. Thrombos Haemostas 1991; 65: 25W-262 beschrieben wurde, in einer Konzentration von 50 µg/ml zugesetzt. Bereits 30 min nach Zusatz des rekombinanten Tick-Anticoagulant-Peptides konnte mit dem chromogenen Substrat keine Enzymaktivität mehr gemessen werden. Der Versuch zeigt, daß partielle Prothrombinase ein effizienter Antagonist der Peptidantikoagulantien, Hirudin und Tick-Anticoagulant Peptide ist.

## Patentansprüche

1. Pharmazeutisches Gerinnungsfaktor-Präparat zur Behandlung von Blutgerinnungsstörungen enthaltend mindestens 2 hochgereinigte einzelne Gerinnungsfaktoren, die Bestandteile einer Prothrombinase oder Pro-Prothrombinase und frei von Phospholipiden sind, wobei die Gerinnungsfaktoren ausgesucht sind aus der Gruppe bestehend aus den Faktoren II, V, Va, X und Xa.

2. Präparat nach Anspruch 1, **dadurch gekennzeichnet, daß** mindestens einer der Faktoren aktiviert ist.

3. Präparat nach Anspruch 1 oder 2, **dadurch gekennzeichnet, daß** es im wesentlichen aus den Faktoren II und V bzw. Va besteht.

4. Präparat nach Anspruch 1 oder 2, **dadurch gekennzeichnet, daß** es im wesentlichen aus den Faktoren X und V bzw. Va besteht.

5. Präparat nach Anspruch 1 oder 2, **dadurch gekennzeichnet, daß** es im wesentlichen aus den Faktoren II und X bzw. Xa, gegebenenfalls in Kombination mit Faktor V bzw. Va besteht.

6. Präparat nach einem der Ansprüche 1 bis 5, **dadurch gekennzeichnet, daß** die Gerinnungsfaktoren als Komplex vorliegen.

7. Präparat nach einem der Ansprüche 1 bis 6, **dadurch gekennzeichnet, daß** es Magnesiumionen enthält.

8. Präparat nach einem der Ansprüche 1 bis 7, **dadurch gekennzeichnet, daß** es keine freien Kalziumionen enthält.

9. Präparat nach Anspruch 8, **dadurch gekennzeichnet, daß** es einen Chelatbildner zum Komplexieren von freien Kalziumionen enthält.

10. Präparat nach einem der Ansprüche 1, 2, und 5 bis 9, **dadurch gekennzeichnet, daß** es Prothrombin und gereinigten Faktor Xa als aktiven Komponenten enthält und frei von Phospholipiden ist.

11. Präparat nach Anspruch 10, **dadurch gekennzeichnet, daß** es im wesentlichen aus Prothrombin und Faktor Xa besteht.

12. Präparat nach Anspruch 10 oder 11, **dadurch gekennzeichnet, daß** es bei einer Dosis von mindestens 150 E Prothrombin/kg frei an thromboembolischen Nebenwirkungen, ausgedrückt durch einen Score im Wessler-Thrombosemodell von höchstens 3 Punkten, ist.

13. Präparat nach Anspruch 10 bis 12, **dadurch gekennzeichnet, daß** es Prothrombin mit einer spezifischen Aktivität von mindestens 5 E/mg Protein enthält.

14. Präparat nach einem der Ansprüche 10 bis 13, **dadurch gekennzeichnet, daß** es Faktor Xa mit einer spezifischen Aktivität von mindestens 100 E/mg Protein enthält.

15. Präparat nach einem der Ansprüche 10 bis 14, **dadurch gekennzeichnet, daß** es Faktor Xa vorwiegend als Faktor Xa β enthält.

16. Präparat nach einem der Ansprüche 10 bis 15, **dadurch gekennzeichnet, daß** Prothrombin und Faktor Xa als Komplex vorliegen.

17. Präparat nach einem der Ansprüche 1 bis 16, **dadurch gekennzeichnet, daß** es weiters Antithrombin III in stabilisierenden Mengen, gegebenenfalls gemeinsam mit Heparin, enthält.

18. Präparat nach einem der Ansprüche 10 bis 17, **dadurch gekennzeichnet, daß** es weniger als 0,1 E Faktor VIII:C oder Faktor VIII:Ag/E Prothrombin enthält.

19. Präparat nach einem der Ansprüche 10 bis 18, **dadurch gekennzeichnet, daß** es weniger als 0,1 E Faktor IX/E Prothrombin enthält.

20. Präparat nach einem der Ansprüche 10 bis 19, **dadurch gekennzeichnet, daß** es weniger als 0,1 E Faktor X/E Prothrombin enthält.

21. Präparat nach einem der Ansprüche 10 bis 20, **dadurch gekennzeichnet, daß** es weniger als 0,01 mg Phospholipide/E Prothrombin enthält.

22. Präparat nach einem der Ansprüche 10 bis 21 in einer Darreichungsform, in einer Konzentration, die eine Dosis von mindestens 50 E Prothrombin/kg Körpergewicht ermöglicht und gegebenenfalls die Administration als Bolus-Injektion erlaubt.

23. Präparat nach Anspruch 22 in einer Darreichungsform, welche eine Dosis von 50 bis 500 E Prothrombin/kg Körpergewicht umfaßt.

24. Präparat nach einem der Ansprüche 1 bis 23, **dadurch gekennzeichnet, daß** die Gerinnungsfaktoren durch rekombinante DNA-Technologie hergestellt sind.

25. Präparat nach einem der Ansprüche 1 bis 24, **dadurch gekennzeichnet, daß** es als Lyophilisat vorliegt, welches vorzugsweise zu einer optisch klaren Lösung rekonstituiert werden kann.

26. Präparat nach einem der Ansprüche 1 bis 25, **dadurch gekennzeichnet, daß** es in einer geeigneten Applikationsvorrichtung vorliegt, vorzugsweise als Lyophilisat in einer Spritze, welche eine in situ-Rekonstitution mit einer pharmazeutisch akzeptablen Lösung erlaubt.

27. Präparat nach einem der Ansprüche 1 bis 26, **dadurch gekennzeichnet, daß** es virusinaktiviert bzw. virusabgereichert ist.

28. Komplex, bestehend aus Prothrombin, Faktor Xa und gegebenenfalls Faktor V bzw. Va.

29. Komplex, bestehend aus Prothrombin und Faktor V bzw. Va.

30. Komplex, bestehend aus Faktor X und Faktor V bzw. Va.

31. Präparat, enthaltend einen Komplex nach einem der Ansprüche 28 bis 30 und weiters Calciumionen.

32. Diagnostisches Präparat nach Anspruch 31, **dadurch gekennzeichnet, daß** es weiters Thrombin und gegebenenfalls Phospholipide enthält, zur diagnostischen Verwendung.

33. Verwendung von gereinigten Prothrombinase-Faktoren, insbesondere von gereinigtem Prothrombin und gereinigtem Faktor Xa, zur Herstellung eines pharmazeutischen Präparates gemäß einem der Ansprüche 1 bis 27 zur Etablierung von supranormalen Prothrombin-Konzentrationen im Blut eines Patienten.

34. Verwendung nach Anspruch 33, wobei die supranormale Konzentration mindestens 1,5 E Prothrombin/ml Blut, vorzugsweise mindestens 2 E/ml, beträgt.

35. Verwendung von gereinigten Prothrombinase-Faktoren, insbesondere von gereinigtem Prothrombin und gereinigtem Faktor Xa, zur Herstellung eines pharmazeutischen Präparates gemäß einem der Ansprüche 1 bis 27 zur Behandlung von Faktor VIII-Inhibitorzuständen, Hämophilie A oder B oder von Willebrand-Krankheit.

36. Verwendung von mindestens 2 Gerinnungsfaktoren, die Bestandteile einer Prothrombinase bzw. Pro-Prothrombinase sind, zur Herstellung einer pharmazeutischen Präparation gemäß einem der Ansprüche 1 bis 27 zur Behandlung von akuten Blutungen, einer gesteigerten Blutungsneigung bzw. einem erhöhten Blutungsrisiko bei nicht-haemophilen Patienten.

37. Verwendung nach Anspruch 36 zur Herstellung einer pharmazeutischen Präparation zur Behandlung von Blutungen aufgrund einer Thrombopathie.

38. Verwendung nach Anspruch 36 oder 37 zur Herstellung einer pharmazeutischen Präparation zur Behandlung einer Heparin-induzierten Thrombozytopänie.

39. Verwendung nach einem der Ansprüche 36 bis 38, **dadurch gekennzeichnet, daß** die pharmazeutische Präparation eine primäre hämostatische Aktivität aufweist.

40. Pharmazeutische Präparation gemäß einem der Ansprüche 1 bis 27 zur Behandlung von Patienten mit Blutgerinnungsstörungen, weiters enthaltend ein Protein mit primärer hämostatischer Aktivität, insbesondere vWF.

41. Set zur Behandlung von Patienten mit Blutgerinnungsstörungen umfassend die Komponenten
a) eine pharmazeutische Präparation gemäß einem der Ansprüche 1 bis 27 und
b) ein Protein mit primärer hämostatischer Aktivität, insbesondere vWF.

42. Verwendung nach einem der Ansprüche 36 bis 38 zur Herstellung einer pharmazeutischen Präparation zur Behandlung von Blutungen im Rahmen einer Antikoagulantien-Therapie.

43. Verwendung nach Anspruch 42 zur Herstellung einer pharmazeutischen Präparation als Antidot für einen Gerinnungsinhibitor bzw. für ein Antikoagulans oder für einen Thrombozytenfunktionshemmer.

44. Verwendung nach Anspruch 43, **dadurch gekennzeichnet, daß** der Gerinnungsinhibitor bzw. das Antikoagulans ausgewählt ist aus der Gruppe bestehend aus APAP, Benzamidinderivat, Hirudin, Heparin, Heparinanaloga, insbesondere Pentasaccharide, AT III-Heparinkomplex, AT III, Antistasin, "Tick-Anticoagulant Peptide", inaktive Gerinnungsfaktoren, insbesondere "active site" inhibierte Gerinnungsfaktoren, TFPI, kompetitive Liganden für Thrombozytenmembranoberflächenrezeptoren, insbesondere Antikörper gegen GP IIb/IIIa, und deren gentechnisch oder synthetisch hergestellten Analoga, insbesondere Peptide, sowie orale Antikoagulantien.

45. Verwendung nach Anspruch 43, **dadurch gekennzeichnet, daß** der Thrombozytenfunktionshemmer Ticlopidin oder Acetylsalicylsäure ist.

46. Set zur Antikoagulantien-Therapie von Patienten umfassend die Komponenten
a) ein Gerinnungsinhibitor bzw. Antikoagulans oder ein Thrombozytenfunktionshemmer, und
b) eine pharmazeutische Präparation nach einem der Ansprüche 1 bis 32.

47. Verwendung nach Anspruch 36 zur Herstellung einer pharmazeutischen Präparation zur Behandlung von intracraniellen Blutungen, insbesondere bei gestörter Thrombingenerierung.

48. Verwendung nach Anspruch 47 zur Behandlung von Frühgeborenen.

## Claims

1. A pharmaceutical coagulation-factor preparation for the treatment of blood coagulation disorders comprising at least 2 highly purified single coagulation factors which are components of a prothrombinase or of a pro-prothrombinase and free from phospholipids, the coagulation factors being selected from the group consisting of factors II, V, Va, X and Xa.

2. A preparation according to claim 1, **characterised in that** at least one of the factors is activated.

3. A preparation according to claim 1 or 2, **characterised in that** it is substantially comprised of factors II and V, or Va, respectively.

4. A preparation according to claim 1 or 2, **characterised in that** it is substantially comprised of factors X and V, or Va, respectively.

5. A preparation according to claim 1 or 2, **characterised in that** it is substantially comprised of factors II and X, or Xa, respectively, optionally in combination with factor V, or Va, respectively.

6. A preparation according to any one of claims 1 to 5, **characterised in that** the coagulation factors are present as a complex.

7. A preparation according to any one of claims 1 to 6, **characterised in that** it contains magnesium ions.

8. A preparation according to any one of claims 1 to 7, **characterised in that** it does not contain any free calcium ions.

9. A preparation according to claim 8, **characterised in that** it contains a chelating agent for complexing free calcium ions.

10. A preparation according to any one of claims 1, 2 and 5 to 9, **characterised in that** it contains prothrombin and purified factor Xa as active components and is free from phospholipids.

11. A preparation according to claim 10, **characterised in that** it is essentially comprised of prothrombin and factor Xa.

12. A preparation according to claim 10 or 11, **characterised in that** at a dose of at least 150 U of prothrombin/kg it is free from thromboembolic side effects, expressed by a score of 3 at the most in the Wessler thrombosis model.

13. A preparation according to claim 10 to 12, **characterised in that** it comprises prothrombin at a specific activity of at least 5 U/mg protein.

14. A preparation according to any one of claims 10 to 13, **characterised in that** it comprises factor Xa at a specific activity of at least 100 U/mg protein.

15. A preparation according to any one of claims 10 to 14, **characterised in that** it comprises factor Xa primarily as factor Xaβ.

16. A preparation according to any one of claims 10 to 15, **characterised in that** prothrombin and factor Xa are present as a complex.

17. A preparation according to any one of claims 1 to 16, **characterised in that** it further comprises antithrombin III in stabilizing amounts, optionally together with heparin.

18. A preparation according to any one of claims 10 to 17, **characterised in that** it comprises less than 0.1 U factor VIII:C or factor VIII:Ag/U prothrombin.

19. A preparation according to any one of claims 10 to 18, **characterised in that** it comprises less than 0.1 U factor IX/U prothrombin.

20. A preparation according to any one of claims 10 to 19,
**characterised in that** it comprises less than 0.1 U factor X/U prothrombin.

21. A preparation according to any one of claims 10 to 20, **characterised in that** it comprises less than 0.01 mg phospholipids/U prothrombin.

22. A preparation according to any one of claims 10 to 21 in an administrable form at a concentration which enables a dose of at least 50 U prothrombin/kg body weight and optionally allows for the administration as a bolus injection.

23. A preparation according to claim 22 in an administrable form which comprises a dose of from 50 to 500 U prothrombin/kg body weight.

24. A preparation according to any one of claims 1 to 23, **characterised in that** the coagulation factors are prepared by recombinant DNA technology.

25. A preparation according to any one of claims 1 to 24, **characterised in that** it is provided as a lyophilisate which preferably can be reconstituted into an optically clear solution.

26. A preparation according to any one of claims 1 to 25, **characterised in that** it is provided in a suitable application device, preferably as a lyophilisate in a syringe which allows for an in situ reconstitution with a pharmaceutically acceptable solution.

27. A preparation according to any one of claims 1 to 26, **characterised in that** it is virus-inactivated, or virus-depleted, respectively.

28. A complex comprised of prothrombin, factor Xa and optionally factor V, or Va, respectively.

29. A complex comprised of prothrombin and factor V, or Va, respectively.

30. A complex comprised of factor X and factor V, or Va, respectively.

31. A preparation comprising a complex according to any one of claims 28 to 30, and furthermore calcium ions.

32. A diagnostic preparation according to claim 31, **characterised in that** it further comprises thrombin and optionally phospholipids, for diagnostic use.

33. The use of purified prothrombinase factors, in particular of purified prothrombin and purified factor Xa, for preparing a pharmaceutical preparation according to any one of claims 1 to 27 to establish supranormal prothrombin concentrations in a patient's blood.

34. The use according to claim 33, wherein the supranormal concentration amounts to at least 1.5 U of prothrombin/ml blood, preferably at least 2 U/ml.

35. The use of purified prothrombinase factors, in particular of purified prothrombin and purified factor Xa, for preparing a pharmaceutical preparation according to any one of claims 1 to 27 for treating factor VIII inhibitor conditions, hemophilia A or B or von Willebrand Disease.

36. The use of at least 2 coagulation factors which are components of a prothrombinase or of a pro-prothrombinase, respectively, for preparing a pharmaceutical preparation according to any one of claims 1 to 27 for treating acute bleedings, an increased bleeding propensity or an increased bleeding risk in non-hemophiliac patients.

37. The use according to claim 36 for preparing a pharmaceutical preparation for treating bleedings caused by a thrombopathy.

38. The use according to claim 36 or 37 for preparing a pharmaceutical preparation for treating a heparin-induced thrombocytopenia.

39. The use according to any one of claims 36 to 38, **characterised in that** the pharmaceutical preparation comprises a primary hemostatic activity.

40. A pharmaceutical preparation according to any one of claims 1 to 27 for treating patients suffering from blood coagulation disorders, and further comprising a protein having primary hemostatic acitivity, in particular vWF.

41. A kit for treating patients suffering from blood coagulation disorders, comprising the components
a) a pharmaceutical preparation according to any one of claims 1 to 27, and
b) a protein having primary hemostatic acitivity, in particular vWF.

42. The use according to any one of claims 36 to 38 for preparing a pharmaceutical preparation for treating bleedings in the course of an anticoagulant therapy.

43. The use according to claim 42 for preparing a pharmaceutical preparation as an antidote for a coagulation inhibitor or for an anticoagulant, respectively, or for an inhibitor of thrombocyte function.

44. The use according to claim 43, **characterised in that** the coagulation inhibitor, or the anticoagulant, respectively, is selected from the group consisting of APAP, benzamidine derivative, hirudin, heparin, heparin analogues, in particular pentasaccharides, AT III heparin complex, AT III, antistasin, tick-anticoagulant peptide, inactive clotting factors, in particular active site inhibited clotting factors, TFPI, competitive ligands for thrombocyte membrane surface receptors, in particular antibodies to GP IIb/IIIa, and their genetically engineered or synthetically produced analogues, in particular peptides, as well as oral anticoagulants.

45. The use according to claim 43, **characterised in that** the inhibitor of thrombocyte function is ticlopidin or acetyl salicylic acid.

46. A kit for anticoagulant therapy of patients, comprising the components
a) a coagulation inhibitor, or an anticoagulant, respectively, or a thrombocyte function inhibitor, and
b) a pharmaceutical preparation according to any one of claims 1 to 32.

47. The use according to claim 36 for preparing a pharmaceutical preparation for treating intracranial bleedings, in particular in case of impaired thrombin generation.

48. The use according to claim 47 for treating premature infants.

## Revendications

1. Préparation pharmaceutique à base de facteurs de coagulation pour le traitement des troubles de la coagulation sanguine, comprenant au moins deux facteurs de coagulation individuels hautement purifiés, qui sont des composants d'une prothrombinase ou d'une pro-prothrombinase et qui sont exempts de phospholipides, les facteurs de coagulation étant choisis parmi l'ensemble constitué par les facteurs II, V, Va, X et Xa.

2. Préparation selon la revendication 1, **caractérisée en ce qu'**au moins l'un des facteurs est activé.

3. Préparation selon la revendication 1 ou 2, **caractérisée en ce qu'**elle est constituée essentiellement des facteurs II et V ou Va.

4. Préparation selon la revendication 1 ou 2, **caractérisée en ce qu'**elle est constituée essentiellement des facteurs X et V ou Va.

5. Préparation selon la revendication 1 ou 2, **caractérisée en ce qu'**elle est constituée essentiellement des facteurs II et X ou Xa, éventuellement en combinaison avec le facteur V ou Va.

6. Préparation selon l'une quelconque des revendications 1 à 5, **caractérisée en ce que** les facteurs de coagulation se présentent sous forme de complexe.

7. Préparation selon l'une quelconque des revendications 1 à 6, **caractérisée en ce qu'**elle contient des ions magnésium.

8. Préparation selon l'une quelconque des revendications 1 à 7, **caractérisée en ce qu'**elle ne contient pas d'ions calcium libres.

9. Préparation selon la revendication 8, **caractérisée en ce qu'**elle contient un agent chélatant pour complexer les ions calcium libres.

10. Préparation selon l'une quelconque des revendications 1, 2 et 5 à 9, **caractérisée en ce qu'**elle contient de la prothrombine et le facteur Xa purifié comme composants actifs et qu'elle est exempte de phospholipides.

11. Préparation selon la revendication 10, **caractérisée en ce qu'**elle est constituée essentiellement de prothrombine et de facteur Xa.

12. Préparation selon la revendication 10 ou 11, **caractérisée en ce que**, à une dose d'au moins 150 U de prothrombine/kg, elle est dépourvue d'effets secondaires thrombo-emboliques, ce qui s'exprime par un score dans le modèle de thrombose de Wessler, de points au maximum.

13. Préparation selon la revendication 10 à 12, **caractérisée en ce qu'**elle contient une prothrombine présentant une activité spécifique d'au moins 5 U/mg de protéine.

14. Préparation selon l'une quelconque des revendications 10 à 13, **caractérisée en ce qu'**elle contient le facteur Xa doté d'une activité spécifique d'au moins 100 U/mg de protéine.

15. Préparation selon l'une quelconque des revendications 10 à 14, **caractérisée en ce qu'**elle contient le facteur Xa, principalement sous la forme du facteur Xa β.

16. Préparation selon l'une quelconque des revendications 10 à 15, **caractérisée en ce que** la prothrombine et le facteur Xa se présentent sous la forme d'un complexe.

17. Préparation selon l'une quelconque des revendications 1 à 16, **caractérisée en ce qu'**elle contient en outre l'antithrombine III en quantités stabilisantes, éventuellement conjointement avec de l'héparine.

18. Préparation selon l'une quelconque des revendications 10 à 17, **caractérisée en ce qu'**elle contient moins de 0,1 U de facteur VIII:C ou de facteur VIII:Ag pour 1 U de prothrombine.

19. Préparation selon l'une quelconque des revendications 10 à 18, **caractérisée en ce qu'**elle contient moins de 0,1 U de facteur IX pour 1 U de prothrombine.

20. Préparation selon l'une quelconque des revendications 10 à 19, **caractérisée en ce qu'**elle contient moins de 0,1 U de facteur X pour 1 U de prothrombine.

21. Préparation selon l'une quelconque des revendications 10 à 20, **caractérisée en ce qu'**elle contient moins de 0,01 mg de phospholipide pour 1 U de prothrombine.

22. Préparation selon l'une quelconque des revendications 10 à 21 sous une forme galénique, selon une concentration qui permet une dose d'au moins 50 U de prothrombine/kg de poids corporel et éventuellement l'administration par bolus/injection.

23. Préparation selon la revendication 22, sous une forme galénique qui comprend une dose de 50 à 500 U de prothrombine/kg de poids corporel.

24. Préparation selon l'une quelconque des revendications 1 à 23, **caractérisée en ce que** les facteurs de coagulation sont produits par la technologie de l'ADN recombinant.

25. Préparation selon l'une quelconque des revendications 1 à 24, **caractérisée en ce qu'**elle se présente sous la forme d'un lyophilisat qui peut être reconstitué de préférence en une solution optiquement limpide.

26. Préparation selon l'une quelconque des revendications 1 à 25, **caractérisée en ce qu'**elle se présente dans un dispositif d'application approprié, de préférence sous la forme d'un lyophilisat dans une seringue, qui permet une reconstitution in situ avec une solution pharmaceutiquement acceptable.

27. Préparation selon l'une quelconque des revendications 1 à 26, **caractérisée en ce qu'**elle inactivée en virus ou appauvrie en virus.

28. Complexe constitué de prothrombine, de facteur Xa et éventuellement de facteur V ou Va.

29. Complexe constitué de prothrombine et de facteur V ou Va.

30. Complexe constitué de facteur X et de facteur V ou Va.

31. Préparation contenant un complexe selon l'une quelconque des revendications 28 à 30 et en outre des ions calcium.

32. Préparation pour diagnostic selon la revendication 31, **caractérisée en ce qu'**elle contient en outre de la thrombine et éventuellement des phospholipides pour une utilisation diagnostique.

33. Utilisation de prothombinase/facteurs purifiés, en particulier de prothrombine purifiée et de facteur Xa purifié, pour la production d'une préparation pharmaceutique selon l'une quelconque des revendications 1 à 27, pour l'établissement de concentrations de prothrombine supranormales dans le sang d'un patient.

34. Utilisation selon la revendication 33, où la concentration supranormale est au moins de 1,5 U de prothrombine/ml de sang, de préférence au moins de 2 U/ml.

35. Utilisation de prothrombinase/facteurs purifiés, en particulier de prothrombine purifiée et de facteur Xa purifié pour la production d'une préparation pharmaceutique selon l'une quelconque des revendications 1 à 27 pour le traitement des états inhibiteurs du facteur VIII, le traitement de l'hémophilie A ou B ou le traitement de la maladie de von Willebrand.

36. Utilisation d'au moins 2 facteurs de coagulation qui sont les composants d'une prothrombinase ou d'une pro-prothrombinase pour la production d'une préparation pharmaceutique selon l'une quelconque des revendications 1 à 27 pour le traitement des hémorragies aiguës, d'une tendance hémorragique accrue ou d'un risque hémorragique accru chez les patients non hémophiles.

37. Utilisation selon la revendication 36 pour la production d'une préparation pharmaceutique pour le traitement des hémorragies dues à une thrombopathie.

38. Utilisation selon la revendication 36 ou 37 pour la production d'une préparation pharmaceutique pour le traitement d'une thrombopénie induite par l'héparine.

39. Utilisation selon l'une quelconque des revendications 36 à 38, **caractérisée en ce que** la préparation pharmaceutique présente une activité hémostatique primaire.

40. Préparation pharmaceutique selon l'une quelconque des revendications 1 à 27, pour le traitement de patients présentant des troubles de la coagulation, contenant en outre une protéine présentant une activité hémostatique primaire, en particulier vWF (facteur de von Willebrand).

41. Kit de traitement de patients présentant des troubles de la coagulation, ledit kit comprenant les composants :
a) une préparation pharmaceutique selon l'une quelconque des revendications 1 à 27 et
b) une protéine présentant une activité hémostatique, en particulier vWF.

42. Utilisation selon l'une quelconque des revendications 36 à 38 pour la production d'une préparation pharmaceutique pour le traitement des hémorragies dans le cadre d'une thérapie anticoagulante.

43. Utilisation selon la revendication 42, pour la production d'une préparation pharmaceutique comme antidote d'un inhibiteur de la coagulation, notamment d'un anticoagulant, ou d'un inhibiteur de la fonction thrombocytaire.

44. Utilisation selon la revendication 43, **caractérisée en ce que** l'inhibiteur de coagulation ou l'anticoagulant est choisi parmi l'ensemble constitué par l'ADAP, un dérivé de benzamidine, l'hirudine, l'héparine, les analogues de l'héparine, en particulier un pentasaccharide, le complexe héparine-AT III, l'AT III, l'antistasine, le «Tick-Anticoagulant Peptide», les facteurs de coagulation inactifs, en particulier les facteurs de la coagulation à "site(s) actif(s) inhibé(s), le TFPI, les ligands compétitifs pour les récepteurs membranaires de surface des thrombocytes, en particulier les anticorps contre GP IIb/IIIa et leurs analogues produits par génie génétique ou de manière synthétique, en particulier les peptides, ainsi que des anticoagulants oraux.

45. Utilisation selon la revendication 43, **caractérisée en ce que** l'inhibiteur de la fonction thrombocytaire est la ticlopidine ou l'acide acétylsalicylique.

46. Kit de thérapie anticoagulante de patients, comprenant les composants :
a) un inhibiteur de la coagulation, notamment un anticoagulant, ou un inhibiteur de la fonction thrombocytaire, et
b) une préparation pharmaceutique selon l'une quelconque des revendications 1 à 32.

47. Utilisation selon la revendication 36 pour la production d'une préparation pharmaceutique pour le traitement des hémorragies intracrâniennes en cas de troubles de la production de thrombine.

48. Utilisation selon la revendication 47 pour le traitement de prématurés.
